# EUROPEAN PATENT SPECIFICATION

(11) **EP 0 512 948 B1**
(45) Date of publication and mention of the grant of the patent: **28.08.1996**
(21) Application number: 92810300.1
(22) Date of filing: 27.04.1992
(51) Int. Cl.: C07D 211/94, C07D 211/46, C07D 211/58, C08K 5/3435

(54) **Substituted 1-hydroxy-2,6-Di-aryl-4-acyloxypiperidines or 1-hydroxy-2,6-diaryl-4-acyl-aminopiperidines and stabilized compositions**
1-Hydroxy-2,6-Diaryl-4-Acyloxypiperidine oder 1-Hydroxy-2,6-Diaryl-4-Acylaminopiperidine und stabilisierte Mischungen
1-Hydroxy-2,6-diaryl-4-acyloxypipéridines ou 1-hydroxy-2,6-diaryl-4-acylaminopipéridines et compositions stabilisées

(30) Priority: 07.05.1991 US 696695
(43) Date of publication of application: 11.11.1992
(73) Proprietor: CIBA-GEIGY AG, 4002 Basel (CH)
(72) Inventor: Cunkle, Glen T., Stamford, CT 06906 (US); Ravichandran, Ramanathan, Nanuet, NY 10954 (US); Sabrsula, Donald J., Peekskill, NY 10566 (US)

(56) References cited:
- EP-A- 0 309 401
- US-A- 4 316 996
- J. POLY. SCI. POLYMER LETTERS ED. vol. 23, 1985, page 475; H. KOTHANDRARAMAN ET. AL.:
- TETRAHEDRON LETTERS. vol. 32, no. 31, 29 July 1991, OXFORD GB pages 3873 - 3874; A. RAJANARAYANAN ET. AL.: 'DEACETYLATION OF N-ACETYLPIPERIDIN-4-ONES BY A NOVEL ELECTROCHEMICAL METHOD.'
- CHEMICAL ABSTRACTS, vol. 100, 1984, Columbus, Ohio, US; abstract no. 120386, NANJAPPAN ET. AL.: 'CARBON-13-SHIFTS IN SUBSTITUTED 1-HETERA-2,6-DIARYL-4- CYCLOHEXANOL ACETATES.' page 531 ;

## Description

The present invention pertains to novel substituted 1-hydroxy-2,6-diaryl-4-acyloxypiperidines or 1-hydroxy-2,6-diaryl-4-acylaminopiperidines and their use as stabilizers for various organic materials subject to the deleterious effects of oxygen, heat or actinic radiation. The instant compounds provide both melt flow stabilization and good color retention during processing as well as good retention of polymer physical properties during long-term thermooxidative stress.

### Background of the Invention

U.S. Patent No. 4,316,996 teaches that 1-hydroxy-2,6-dialkylpiperidines are useful in preventing the discoloration of phenolic antioxidants.

1-Hydroxy-2,2,6,6-tetramethyl-4-acyloxypiperidines and other 1-hydroxy-2,2,6,6-tetramethylpiperidine derivatives are known as effective light stabilizers, but these compounds are not effective as processing stabilizers. They are also structurally distinct from the instant compounds of this invention.

U.S. Patent Nos. 4,668,721; 4,782,105; 4,876,300 and 4,898,901 describe other hydroxylamines which are useful as processing stabilizers, but these hydroxylamines are structurally quite different from the instant compounds.

H. Kothandraraman et al., J. Poly. Sci. Polymer Letters Ed., 23, 475 (1985) report the preparation of polyacrylamides having the amino group substituted by selected substituted 2,6-diarylpiperidin-4-yl moieties. The instant compounds and their utility as stabilizers are not mentioned or suggested by this scientific article.

The instant 1-hydroxy-2,6-diaryl-4-acyloxypiperidines and 1-hydroxy-2,6-diaryl-4-acylaminopiperidines are novel compounds unknown in the prior art. The precursor amine starting materials are also novel compounds.

The 1-hydroxy-2,6-diaryl-4-acyloxypiperidines and 1-hydroxy-2,6-diaryl-4-acylaminopiperidines of this invention exhibit surprisingly superior properties compared to those of the closest prior art compounds.

The instant compounds are structurally distinct from prior art compounds. Their structures allow for the synthesis of high molecular weight compounds which have low volatility, better compatibility with the substrate and low extractability. The instant compounds provide both melt flow stabilization and good resistance against discoloration during polymer processing. The instant compounds have superior hydrolytic stability over the state of the art phosphite stabilizers and exhibit superior long term heat aging and oxidative induction time over the state of the art hydroxylamine stabilizers.

### Objects of the Invention

One object of this invention is to provide new substituted 1-hydroxy-2,6-diaryl-4-acyloxypiperidines or new 1-hydroxy-2,6-diaryl-4-acylaminopiperidines which are efficacious stabilizers for organic materials subject to oxidative, thermal and/or actinic degradation.

Another object of the invention is to provide stabilized compositions containing the substituted 1-hydroxy-2,6-diaryl-4-acyloxy- piperidines or 1-hydroxy-2,6-diaryl-4-acylaminopiperidines of this invention.

Still another object of this invention is to provide new substituted 2,6-diaryl-4-acyloxypiperidines or 2,6-diaryl-4-acylaminopiperidines which are useful as starting materials for making the 1-hydroxy-2,6-diaryl-4-acyloxypiperidine or 1-hydroxy-2,6-diaryl-4-acylaminopiperidine stabilizers of this invention.

### Detailed Disclosure

The instant invention pertains to novel substituted 1-hydroxy-2,6-diaryl-4-acyloxypiperidines or 1-hydroxy-2,6-diaryl-4-acylaminopiperidines of formula I 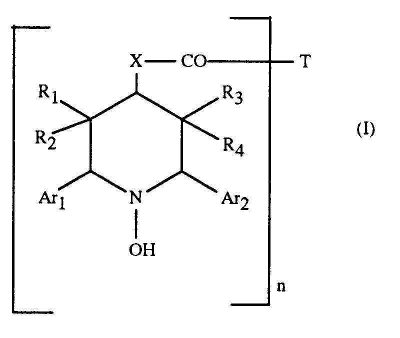 wherein
n is 1, 2, 3 or 4;
X is -O- or -NE-,
E is hydrogen, alkyl of 1 to 20 carbon atoms or cycloalkyl of 5 to 12 carbon atoms,
Ar₁ and Ar₂ are independently aryl of 6 to 10 carbon atoms; or said aryl substituted by one to three substituents selected from the group consisting of alkyl of 1 to 20 carbon atoms; cycloalkyl of 5 to 12 carbon atoms; phenylalkyl of 7 to 15 carbon atoms; -COOR₅ where R₅ is hydrogen or alkyl of 1 to 20 carbons; -COR₆ where R₆ is alkyl of 1 to 20 carbons; -NR₇R₈ where R₇ and R₈ are independently hydrogen or alkyl of 1 to 20 carbons; -SR₉ where R₉ is aryl of 6 to 10 carbon atoms or alkyl of 1 to 20 carbon atoms; -OH; -OCH₃; -CN; -CF₃; -NO₂; -F; -Cl; -Br and -I;
R₁, R₂, R₃and R₄ are independently hydrogen; a linear or branched alkyl of 1 to 30 carbon atoms; or said alkyl terminated with -OR₁₀, -NR₁₁R₁₂, -SR₁₃, -COOR₁₄ or -CONR₁₅R₁₆, where R₁₀, R₁₁, R₁₂, R₁₃ and R₁₄ are independently alkyl of 1 to 20 carbon atoms or alkenyl of 3 to 18 carbon atoms, and R₁₅ and R₁₆ are independently hydrogen or the same meaning as R₁₀; or said alkyl interrupted by one or more -O-, -S-, -SO-, -SO₂-, -CO-, -COO-, -OCO-, -CONR₁₇-, -NR₁₇CO- or -NR₁₈- where R₁₇ and R₁₈ have the same meaning as R₁₅; alkenyl of 3 to 20 carbon atoms; aryl of 6 to 10 carbon atoms; or said aryl substituted by one to three substituents selected from the group consisting of alkyl of 1 to 20 carbon atoms, cycloalkyl of 5 to 12 carbon atoms, and phenylalkyl of 7 to 15 carbon atoms; and
when n is 1, T is alkyl of 1 to 20 carbon atoms or said alkyl interrupted by one or more of -O-, -S-, -SO-, -SO₂-, -CO-, -COO-, -OCO-, -CONR₁₉-,-NR₁₉CO- or -NR₂₀- where R₁₉ and R₂₀ have the same meaning as R₁₅; or aryl or substituted aryl having the same definition as Ar₁;
when n is 2, T is a direct bond; alkylene of 1 to 12 carbon atoms, or said alkylene interrupted by one or more of -O-, -S-, -SO-, -SO₂-, -CO-, -COO-, -OCO-, -CONR₂₁, -NR₂₁CO- or -NR₂₂- where R₂₁ and R₂₂ have the same meaning as R₁₅;
when n is 3, T is alkanetriyl of 3 to 8 carbon atoms; and
when n is 4, T is alkanetetrayl of 4 to 10 carbon atoms.

All possible geometric isomers and stereoisomers which are predictable are to be included in the scope of this invention.

Preferably, n is 1 or 2.

Preferably X is -O-.

Preferably, Ar₁ and Ar₂ are the same and each is phenyl or phenyl substituted by methyl. Most preferably Ar₁ and Ar₂ are phenyl.

Preferably, when n is 1, T is alkyl of 1 to 17 carbon atoms; most preferably 7 to 17 carbon atoms.

Preferably, when n is 2, T is alkylene of 2 to 10 carbon atoms; most preferably 2 to 8 carbon atoms.

Preferably, R₁, R₂, R₃ and R₄ are independently hydrogen or methyl. Most preferably each of R₁, R₂, R₃ and R₄ is hydrogen; or R₁ is methyl, and R₂, R₃ and R₄ are hydrogen; or R₁ and R₃ are methyl, and R₂ and R₄ are hydrogen.

Preferred are compounds of formula I wherein
E is hydrogen or alkyl of 1 to 20 carbon atoms;
Ar₁ and Ar₂ are independently phenyl; or phenyl substituted by one to three substituents selected from the group consisting of alkyl of 1 to 12 carbon atoms; cycloalkyl of 5 to 7 carbon atoms; -COOH; -COO-CH₃; -COO-C₂H₅; -CO-CH₃; -CO-C₂H₅; -NH₂; -OH; and -OCH₃;
R₁, R₂, R₃ and R₄ are independently hydrogen, methyl or ethyl; and
when n is 1, T is alkyl of 1 to 20 carbon atoms or said alkyl interrupted by one or more of -O-, -S-, -SO-, -SO₂-, -CO-, -COO-, -OCO- or phenylene;
when n is 2, T is a direct bond; alkylene of 1 to 12 carbon atoms, or said alkylene interrupted by one or more of -O-, -S-, -SO-, -SO₂-, -CO-, -COO- or -OCO-;
when n is 3, T is alkanetriyl of 3 to 8 carbon atoms; and
when n is 4, T is alkanetetrayl of 4 to 10 carbon atoms.

Most preferred are compounds of formula I wherein n is 1 or 2; E is hydrogen; each of Ar₁ and Ar₂ are phenyl;
each of R₁, R₂, R₃ and R₄ is hydrogen; or R₁ is methyl, and R₂, R₃ and R₄ are hydrogen;
or R₁ and R₃ are methyl, and R₂ and R₄ are hydrogen; and,
when n is 1, T is alkyl of 7 to 17 carbon atoms; and,
when n is 2, T is alkylene of 2 to 10 carbon atoms.

The instant invention also pertains to stabilized compositions containing
(a) an organic material subject to oxidative, thermal or actinic degradation, and
(b) an effective stabilizing amount of a compound of formula I as defined above.

Preferred are compositions wherein the organic material is a synthetic polymer.

The invention therefore includes a method of stabilizing organic materials against oxidative, thermal or actinic degradation, which comprises incorporating into said materials at least one compound of formula I. The invention also includes the use of said compounds as stabilizer, especially as process stabilizer for synthetic polymers.

The oxidation of secondary amines to hydroxylamines using dimethyloxirane has been reported by Murray and Singh, Synthetic Comm. 1989, 19, 3509. The instant compounds are prepared by the oxidation of the corresponding amine with dimethyldioxirane by the procedure of Eaton and Wicks, J. Org. Chem. 1988, 53, 5353. The corresponding amine is conveniently prepared by the acylation of the corresponding 4-hydroxypiperidine or 4-aminopiperidine.

These substituted 4-hydroxypiperidines, such as 2,6-diphenyl-4-hydroxypiperidine, 2,6-diphenyl-3-methyl-4-hydroxypiperidine and 2,6-diphenyl-3,5-dimethyl-4-hydroxypiperidine, are prepared by published procedures, Balasubramanian, M.; Padma, N., Tetrahedron 19, 2135 (1963).

The 2,6-diaryl-4-aminopiperidines are synthesized by oximation of the corresponding ketone as taught by P. Geneste et al., J. Org. Chem. 41, 3437 (1976) and R. Haller et al., Tetrahedron, 28, 2863 (1972); followed by reduction to the primary amine as taught by M. Uma et al., Indian J. Chem., 19B, 74 (1980).

Another aspect of the instant invention is the amine starting materials of formula II 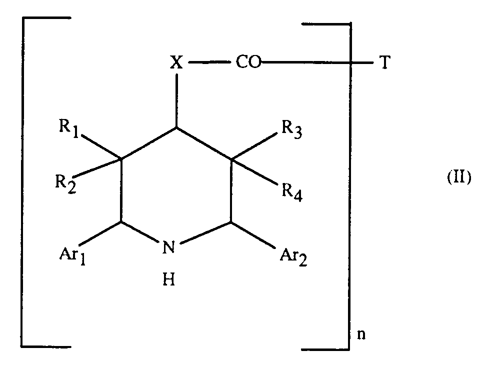 used to prepare the instant compounds of formula I. Ar₁, Ar₂, R₁ to R₂₂, X, E and T in formula II have the same meanings as defined above for formula I. The amine precursor starting material of formula II differs from compounds of formula I in that there is no hydroxyl group present on the N atom in the piperidine ring.

Preferably, in compounds of formula II, n is 1 or 2.

Preferably X is -O-.

Preferably, Ar₁ and Ar₂ are the same and each is phenyl or phenyl substituted by methyl. Most preferably Ar₁ and Ar₂ are phenyl.

Preferably, when n is 1, T is alkyl of 1 to 17 carbon atoms; most preferably 7 to 17 carbon atoms.

Preferably, when n is 2, T is alkylene of 2 to 10 carbon atoms; most preferably 2 to 8 carbon atoms.

Preferably, R₁, R₂, R₃ and R₄ are independently hydrogen or methyl. Most preferably each of R₁, R₂, R₃ and R₄ is hydrogen; or R₁ is methyl, and R₂, R₃ and R₄ are hydrogen; or R₁ and R₃ are methyl, and R₂ and R₄ are hydrogen

Preferred are compounds of formula II wherein
E is hydrogen or alkyl of 1 to 20 carbon atoms;
Ar₁ and Ar₂ are independently phenyl; or phenyl substituted by one to three substituents selected from the group consisting of alkyl of 1 to 12 carbon atoms; cycloalkyl of 5 to 7 carbon atoms; -COOH; -COO-CH₃; -COO-C₂H₅; -CO-CH₃; -CO-C₂H₅; -NH₂; -OH; and -OCH₃;
R₁, R₂, R₃ and R₄ are independently hydrogen, methyl or ethyl; and
when n is 1, T is alkyl of 1 to 20 carbon atoms or said alkyl interruptedby one or more of -O-, -S-, -SO-, -SO₂-, -CO-, -COO-, -OCO- or phenylene;
when n is 2, T is a direct bond; alkylene of 1 to 12 carbon atoms, or said alkylene interrupted by one or more of -O-, -S-, -SO-, -SO₂-, -CO-, -COO- or -OCO-;
when n is 3, T is alkanetriyl of 3 to 8 carbon atoms; and
when n is 4, T is alkanetetrayl of 4 to 10 carbon atoms.

Most preferred are compounds of formula II wherein n is 1 or 2; E is hydrogen; each of Ar₁ and Ar₂ are phenyl;
each of R₁, R₂, R₃ and R₄ is hydrogen; or R₁ is methyl, and R₂, R₃ and R₄ are hydrogen;
or R₁ and R₃ are methyl, and R₂ and R₄ are hydrogen; and,
when n is 1, T is alkyl of 7 to 17 carbon atoms; and,
when n is 2, T is alkylene of 2 to 10 carbon atoms.

When any of Ar₁, Ar₂, R₁ to R₂₂, E or T is alkyl, such alkyl groups are, for example, methyl, ethyl, isopropyl, n-butyl, tert-butyl, tert-amyl, 2-ethylhexyl, n-octyl, n-undecyl, lauryl, n-heptadecyl, n-octadecyl and eicosyl; when said radicals are cycloalkyl, they are, for example, cyclopentyl, cyclohexyl, cyclooctyl and cyclododecyl; when said radicals are phenylalkyl, they are, for example, benzyl, phenethyl, α-methylbenzyl and α,α-dimethylbenzyl; when said radicals are aryl, they are, for example phenyl, naphthyl, or when substituted by alkyl are, for example, tolyl and xylyl; when said radicals are alkyl interrupted by -O-, they are for example, 3-oxaamyl and 3,6-dioxaoctyl; when T is alkyl or said alkyl interrupted by -O-, T is, for example, methylene, ethylene, trimethylene, tetramethylene, octamethylene, 2,2-dimethylpropane-1,3-diyl, 3-oxapentamethylene and 3,6-dioxaoctamethylene; when T is alkanetriyl, it is, for example, glyceryl, trimethylyl propane; and when T is alkanetetrayl, T is, for example, pentaerythrityl or 1,2,3,4-butanetetrayl.

Substrates in which the instant compounds of formula I are particularly useful are polyolefins, such as polyethylene and polypropylene. Polypropylene is particularly well stabilized by the instant compounds during processing.

While the instant compounds of formula I are quite effective process stabilizers when used alone, compositions which also contain a phenolic antioxidant and/or a hindered amine are also extremely well stabilized during processing by this combination of process stabilizers.

In general polymers which can be stabilized include
1. Polymers of monoolefins and diolefins, for example polypropylene, polyisobutylene, polybut-1-ene, polymethylpent-1-ene, polyisoprene or polybutadiene, as well as polymers of cycloolefins, for example of cyclopentene or norbornene, polyethylene (which may be crosslinked), for example high density polyethylene (HDPE), low density polyethylene (LDPE), linear low density polyethylene (LLDPE), branched low density polyethylene (BLDPE).
2. Mixtures of the polymers mentioned in 1), for example mixtures of polypropylene with polyisobutylene, polypropylene with polyethylene (for example PP/HDPE, PP/LDPE) and mixtures of different types of polyethylene (for example LDPE/HDPE).
3. Copolymers of monoolefins and diolefins with each other or with other vinyl monomers, for example ethylene/propylene, linear low density polyethylene (LLDPE) and its mixtures with low density polyethylene (LDPE), propylene/butene-1, ethylene/hexene, ethylene/ethylpentene, ethylene/heptene, ethylene/octene, propylene/isobutylene, ethylene/but-1-ene, propylene/butadiene, isobutylene/isoprene, ethylene/alkyl acrylates, ethylene/alkyl methacrylates, ethylene/vinyl acetate copolymers and their copolymers with carbon monoxide or ethylene/acrylic acid copolymers and their salts (ionomers) and terpolymers of ethylene with propylene and a diene, such as hexadiene, dicyclopentadiene or ethylidene-norbornene; as well as mixtures of such copolymers and their mixtures with polymers mentioned in 1) above, for example polypropylene/ethylene-propylene-copolymers, LDPE/ethylene-vinyl acetate copolymers (EVA), LDPE/ethylene-acrylic acid copolymers (EAA), LLDPE/EVA, LLDPE/EAA and random or alternating polyalkylene/carbon monoxide-copolymers as well as mixtures thereof with other polymers, for example polyamides.
3a. Hydrocarbon resins (for example C₅-C₉) and hydrogenated modifications thereof (for example tackifiers) and mixtures of polyalkylenes and starch.
4. Polystyrene, poly(p-methylstyrene), poly(α-methylstyrene).
5. Copolymers of styrene or α-methylstyrene with dienes or acrylic derivatives, such as, for example, styrene/butadiene, styrene/acrylonitrile, styrene/alkyl methacrylate, styrene/maleic anhydride, styrene/butadiene/alkyl acrylate, styrene/butadiene/alkyl methacrylate styrene/acrylonitrile/methyl acrylate; mixtures of high impact strength from styrene copolymers and another polymer, for example from a polyacrylate, a diene polymer or an ethylene/propylene/diene terpolymer; and block copolymers of styrene, for example styrene/butadiene/styrene, styrene/isoprene/styrene, styrene/ethylene/butylene/styrene or styrene/ethylene/propylene/ styrene.
6. Graft copolymers of styrene or α-methylstyrene, for example styrene on polybutadiene, styrene on polybutadiene-styrene or polybutadiene-acrylonitrile; styrene and acrylonitrile (or methacrylonitrile) on polybutadiene; styrene and maleic anhydride or maleimide on polybutadiene; styrene, acrylonitrile and maleic anhydride or maleimide on polybutadiene; styrene, acrylonitrile and methyl methacrylate on polybutadiene, styrene and alkyl acrylates or methacrylates on polybutadiene, styrene and acrylonitrile on ethylene/propylene/diene terpolymers, styrene and acrylonitrile on polyacrylates or polymethacrylates, styrene and acrylonitrile on acrylate/butadiene copolymers, as well as mixtures thereof with the copolymers listed in 5), for example the copolymer mixtures known as ABS, MBS, ASA or AES polymers.
7. Halogen-containing polymers, such as polychloroprene, chlorinated rubbers, chlorinated or sulfochlorinated polyethylene, copolymers of ethylene and chlorinated ethylene, epichlorohydrin homo- and copolymers, polymers of halogenated vinyl compounds, for example polyvinyl chloride, polyvinylidene chloride, polyvinyl fluoride, polyvinylidene fluoride, as well as copolymers thereof, for example vinyl chloride/vinylidene chloride, vinyl chloride/vinyl acetate or vinylidene chloride/vinyl acetate copolymers.
8. Polymers derived from α,β-unsaturated acids and derivatives thereof, such as polyacrylates and polymethacrylates, polymethyl methacrylate impact-modified with butyl acrylate, polyacrylamides and polyacrylonitriles.
9. Copolymers of the monomers mentioned in 8) with each other or with other unsaturated monomers, for example acrylonitrile/ butadiene, acrylonitrile/alkyl acrylate, acrylonitrile/alkoxyalkyl acrylate or acrylonitrile/vinyl halide copolymers or acrylonitrile/alkyl methacrylate/butadiene terpolymers.
10. Polymers derived from unsaturated alcohols and amines, or acyl derivatives thereof or acetals thereof, such as polyvinyl alcohol, polyvinyl acetate, polyvinyl stearate, polyvinyl benzoate, polyvinyl maleate, polyvinyl butyral, polyallyl phthalate or polyallylmelamine; as well as their copolymers with olefins mentioned in 1) above.
11. Homopolymers and copolymers of cyclic ethers, such as polyalkylene glycols, polyethylene oxide, polypropylene oxide or copolymers thereof with bisglycidyl ethers.
12. Polyacetals, such as polyoxymethylene and those polyoxymethylenes which contain ethylene oxide as comonomer; polyacetals modified with thermoplastic polyurethanes, acrylates or MBS.
13. Polyphenylene oxides and sulfides, and mixtures of polyphenylene oxides with polystyrene or polyamides.
14. Polyurethanes derived from polyethers, polyesters or polybutadienes with terminal hydroxyl groups on the one hand and aliphatic or aromatic polyisocyanates on the other, as well as precursors thereof (polyisocyanates, polyols or prepolymers).
15. Polyamides and copolyamides derived from diamines and dicarboxylic acids and/or from aminocarboxylic acids or the corresponding lactams, such as polyamide 4, polyamide 6, polyamide 6/6, 6/10, 6/9, 6/12, 4/6, 12/12, polyamide 11, polyamide 12, aromatic polyamides obtained by condensation of m-xylene, diamine and adipic acid; polyamides prepared from hexamethylenediamine and isophthalic acid and/or terephthalic acid and optionally an elastomer as modifier, for example poly(2,4,4,-trimethylhexamethylene) terephthalamide or poly-m-phenylene isophthalamide. Further copolymers of the aforementioned polyamides with polyolefins, olefin copolymers, ionomers or chemically bonded or grafted elastomers; or with polyethers, as with polyethylene glycols, polypropylene glycols or polytetramethylene glycols. Polyamides or copolyamides modified with EPDM or ABS. Polyamides condensed during processing (RIM polyamide systems).
16. Polyureas, polyimides and polyamide-imides.
17. Polyesters derived from dicarboxylic acids and diols and/or from hydroxycarboxylic acids or the corresponding lactones, such as polyethylene terephthalate, polybutylene terephthalate, poly-1,4-dimethylolcyclohexane terephthalate, poly[2,2-(4-hydroxyphenyl)propane] terephthalate and polyhydroxybenzoates as well as block-copolyether-esters derived from polyethers having hydroxyl end groups.
18. Polycarbonates and polyester carbonates.
19. Polysulfones, polyether sulfones and polyether ketones.
20. Crosslinked polymers which are derived from aldehydes on the one hand and phenols, ureas and melamines on the other, such as phenol/formaldehyde resins, urea/formaldehyde resins and melamine/formaldehyde resins.
21. Drying and non-drying alkyd resins.
22. Unsaturated polyester resins derived from copolyesters of saturated and unsaturated dicarboxylic acids with polyhydric alcohols and vinyl compounds as crosslinking agents, and also halogenated modifications thereof of low inflammability.
23. Thermosetting acrylic resins derived from substituted acrylic esters, such as epoxy acrylates, urethane acrylates or polyester acrylates.
24. Alkyd resins, polyester resins or acrylate resins in admixture with melamine resins, urea resins, polyisocyanates or epoxy resins as crosslinking agents.
25. Crossfinked epoxy resins which are derived from polyepoxides, for example from bisglycidyl ethers or from cycloaliphatic diepoxides.
26. Natural polymers, such as cellulose, rubber, gelatin and chemically modified homologous derivatives thereof, such as cellulose acetates, cellulose propionates and cellulose butyrates, or the cellulose ethers, such as methyl cellulose; rosins and their derivatives.
27. Mixtures of polymers as mentioned above, for example PP/EPDM, polyamide 6/EPDM or ABS, PVC/EVA, PVC/ABS, PVC/MBS, PC/ABS, PBTP/ABS, PC/ASA, PC/PBT, PVC/CPE, PVC/acrylates, POM/thermoplastic PUR, PC/thermoplastic PUR, POM/acrylate, POM/MBS, PPE/HIPS, PPE/PA 6.6 and copolymers, PA/HDPE, PA/PP, PA/PPE.
28. Naturally occurring and synthetic organic materials which are pure monomeric compounds or mixtures of such compounds, for example mineral oils, animal and vegetable fats, oil and waxes, or oils, fats and waxes based on synthetic esters (e.g. phthalates, adipates, phosphates or trimellithates) and also mixtures of synthetic esters with mineral oils in any weight ratios, which materials may be used as plasticizer for polymers or as textile spinning oils, as well as aqueous emulsions of such materials.
29. Aqueous emulsions of natural or synthetic rubber, e.g. natural latex or latices of carboxylated styrene/butadiene copolymers.
30. Polysiloxanes such as the soft, hydrophilic polysiloxanes described, for example, in U.S. Patent No. 4,259,467; and the hard polyorganosiloxanes described, for example, in U.S. Patent No. 4,355,147.
31. Polyketimines in combination with unsaturated acrylic polyacetoacetate resins or with unsaturated acrylic resins. The unsaturated acrylic resins include the urethane acrylates, polyether acrylates, vinyl or acryl copolymers with pendant unsaturated groups and the acrylated melamines. The polyketimines are prepared from polyamines and ketones in the presence of an acid catalyst.
32. Radiation curable compositions containing ethylenically unsaturated monomers or oligomers and a polyunsaturated aliphatic oligomer.
33. Epoxymelamine resins such as light-stable epoxy resins crosslinked by an epoxy functional coetherified high solids melamine resin such as LSE-4103 (Monsanto).

In general, the compounds of the present invention are employed in from about 0.01 to about 5% by weight of the stabilized composition, although this will vary with the particular substrate and application. An advantageous range is from about 0.05 to about 2%, and especially 0.1 to about 1 %.

The stabilizers of the instant invention may readily be incorporated into the organic polymers by conventional techniques, at any convenient stage prior to the manufacture of shaped articles therefrom. For example, the stabilizer may be mixed with the polymer in dry powder form, or a suspension or emulsion of the stabilizer may be mixed with a solution, suspension, or emulsion of the polymer. The resulting, stabilized polymer compositions of the invention may optionally also contain an other stabilizer, examples of which are listed below, or mixtures thereof.
1. Antioxidants
   1.1. Alkylated monophenols, for example 2,6-di-tert-butyl-4-methyl-phenol, 2-tert-butyl-4,6-dimethylphenol, 2,6-di-tert-butyl-4-ethylphenol, 2,6-di-tert-butyl-4-n-butyl-phenol, 2,6-di-tert-butyl-4-iso-butylphenol, 2,6-dicyclopentyl-4-methylphenol, 2-(α-methylcyclohexyl)-4,6-dimethylphenol, 2,6-dioctadecyl-4-methylphenol, 2,4,6-tricyclohexyl-phenol, 2,6-di-tert-butyl-4-methoxymethylphenol, 2,6-di-nonyl-4-methyl-phenol, 2,4-dimethyl-6-(1′-methyl-undec-1′-yl)-phenol, 2,4-dimethyl-6-(1′-methylheptadec-1′-yl)-phenol, 2,4-dimethyl-6-(1′-methyl-tridec-1′-yl)-phenol and mixtures therof.
   1.2. Alkylthiomethylphenols, for example 2,4-dioctylthiomethyl-6-tert-butylphenol, 2,4-dioctylthiomethyl-6-methylphenol, 2,4-dioctylthiomethyl-6-ethylphenol, 2,6-di-dodecylthiomethyl-4-nonylphenol.
   1.3. Hydroquinones and alkylated hydroquinones, for example 2,6-di-tert-butyl-4-methoxy-phenol, 2,5-di-tert-butylhydroquinone, 2,5-di-tert-amylhydroquinone, 2,6-diphenyl-4-octadecyloxyphenol, 2,6-di-tert-butyl-hydroquinone, 2,5-di-tert-butyl-4-hydroxyanisole, 3,5-di-tert-butyl-4-hydroxyanisole, 3,5-di-tert-butyl-4-hydroxyphenyl-stearate, bis-(3,5-di-tert-butyl-4-hydroxyphenyl)adipate.
   1.4. Hydroxylated thiodiphenyl ethers, for example 2,2′-thiobis(6-tert-butyl-4-methylphenol), 2,2′-thiobis(4-octylphenol), 4,4′-thiobis(6-tert-butyl-3-methylphenol), 4,4′-thiobis(6-tert-butyl-2-methylphenol), 4,4′-thio-bis-(3,6-di-sec-amylphenol), 4,4′-bis-(2,6-dimethyl-4-hydroxyphenyl)-disulfide.
   1.5. Alkylidenebisphenols, for example 2,2′-methylenebis(6-tert-butyl-4-methylphenol), 2,2′-methylenebis(6-tert-butyl-4-ethylphenol), 2,2′-methylenebis[4-methyl-6-(α-methyl-cyclohexyl)phenol], 2,2′-methylenebis(4-methyl-6-cyclohexylphenol), 2,2′-methylenebis(6-nonyl-4-methylphenol), 2,2′-methylenebis(4,6-di-tert-butylphenol), 2,2′-ethylidenebis(4,6-di-tert-butylphenol), 2,2′-ethylidenebis(6-tert-butyl-4-isobutylphenol), 2,2′-methylenebis[6-(α-methylbenzyl)-4-nonylphenol], 2,2′-methylenebis[6-(α,α-dimethylbenzyl)-4-nonylphenol], 4,4′-methylenebis(2,6-di-tert-butylphenol), 4,4′-methylenebis(6-tert-butyl-2-methylphenol), 1,1-bis(5-tert-butyl-4-hydroxy-2-methylphenyl)butane, 2,6-bis(3-tert-butyl-5-methyl-2-hydroxybenzyl)-4-methylphenol, 1,1,3-tris(5-tert-butyl-4-hydroxy-2-methylphenyl)butane, 1,1-bis(5-tert-butyl-4-hydroxy-2-methyl-phenyl)-3-n-dodecylmercaptobutane, ethylene glycol bis[3,3-bis-(3′-tert-butyl-4′-hydroxyphenyl)butyrate], bis(3-tert-butyl-4-hydroxy-5-methyl-phenyl)dicyclopentadiene, bis[2-(3′-tert-butyl-2′-hydroxy-5′-methylbenzyl)-6-tert-butyl-4-methylphenyl]terephthalate, 1,1-bis-(3,5-dimethyl-2-hydroxyphenyl)butan, 2,2-bis-(3,5-di-tert.-butyl-4-hydroxyphenyl)-propan, 2,2-bis-(5-tert.-butyl-4-hydroxy-2-methylphenyl)-4-n-dodecylmercapto-butan, 1,1,5,5-tetra-(5-tert.-butyl-4-hydroxy-2-methylphenyl)-pentan.
   1.6. O-, N- and S-benzyl compounds, for example 3,5,3′,5′-tetra-tert.-butyl-4,4′-dihydroxy-dibenzylether, octadecyl-4-hydroxy-3,5-dimethylbenzyl-mercaptoacetate, tris-(3,5-di-tert.-butyl-4-hydroxybenzyl)-amine, bis-(4-tert.-butyl-3-hydroxy-2,6-dimethylbenzyl)dithioterephthalate, bis-(3,5-di-tert.-butyl-4-hydroxybenzyl)-sulfide, isooctyl-3,5-di-tert.-butyl-4-hydroxybenzyl-mercaptoacetate.
   1.7. Hydroxybenzylated Malonates, for example dioctadecyl-2,2-bis-(3,5-di-tert.-butyl-2-hydroxybenzyl)-malonate, di-octadecyl-2-(3-tert.-butyl-4-hydroxy-5-methylbenzyl)-malonate, di-dodecylmercaptoethyl-2,2-bis-(3,5-di-tert.-butyl-4-hydroxybenzyl)-malonate, Di-[4-(1,1,3,3-tetramethylbutyl)-phenyl]-2,2-bis-(3,5-di-tert.-butyl-4-hydroxybenzyl)-malonate.
   1.8. Hydroxybenzyl-Aromatics, for example 1,3,5-tris-(3,5-di-tert.-butyl-4-hydroxybenzyl)-2,4,6-trimethylbenzene, 1,4-bis-(3,5-di-tert.-butyl-4-hydroxybenzyl)-2,3,5,6-tetramethylbenzene, 2,4,6-tris-(3,5-di-tert.-butyl-4-hydroxybenzyl)-phenol.
   1.9. Triazine Compounds, for example 2,4-bis-octylmercapto-6-(3,5-di-tert.-butyl-4-hydroxyanilino)-1,3,5-triazine, 2-octylmercapto-4,6-bis-(3,5-di-tert.-butyl-4-hydroxyanilino)-1,3,5-triazine, 2-octylmercapto-4,6-bis-(3,5-di-tert.-butyl-4-hydroxyphenoxy)-1,3,5-triazine, 2,4,6-tris-(3,5-di-tert.-butyl-4-hydroxyphenoxy)-1,2,3-triazine, 1,3,5-tris-(3,5-di-tert.-butyl-4-hydroxybenzyl)-isocyanurate, 1,3,5-tris-(4-tert.-butyl-3-hydroxy-2,6-dimethylbenzyl)-isocyanurate, 2,4,6-tris-(3,5-di-tert.-butyl-4-hydroxyphenylethyl)-1,3,5-triazine, 1,3,5-tris-(3,5-di-tert.butyl-4-hydroxyphenylpropionyl)-hexahydro-1,3,5-triazine, 1,3,5-tris-(3,5-dicyclohexyl-4-hydroxybenzyl)-isocyanurate.
   1.10. Benzylphosphonates, for example dimethyl-2,5-di-tert.-butyl-4-hydroxybenzylphosphonate, diethyl-3,5-di-tert.-butyl-4-hydroxybenzylphosphonate, dioctadecyl-3,5-di-tert.-butyl-4-hydroxybenzylphosphonate, dioctadecyl-5-tert.-butyl-4-hydroxy-3-methylbenzylphosphonate, Ca-salt of the 3,5-di-tert.-butyl-4-hydroxybenzyl-phosphonic acid monoethylester.
   1.11. Acylaminophenols, for example lauric acid 4-hydroxyanilide, stearic acid 4-hydroxyanilide, octyl N-(3,5-di-tert-butyl-4-hydroxyphenyl)-carbamate.
   1.12. Esters of β-(3,5-di-tert-butyl-4-hydroxyphenyl)propionic acid with mono- or polyhydric alcohols, e.g,. with methanol, ethanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl) isocyanurate, N,N′-bis(hydroxyethyl)oxalic acid diamide, 3-thiaundecanol, 3-thiapentadecanol, trimethylhexanediol, trimethylolpropane, 4-hydroxymethyl-1-phospha-2,6,7-trioxabicyclo-[2.2.2]-octane.
   1.13. Esters of β-(5-tert-butyl-4-hydroxy-3-methylphenyl)propionic acid with mono- or polyhydric alcohols, e.g,. with methanol, ethanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl) isocyanurate, N,N′-bis(hydroxyethyl)oxalic acid diamide, 3-thiaundecanol, 3-thiapentadecanol, trimethylhexanediol, trimethylolpropane, 4-hydroxymethyl-1-phospha-2,6,7-trioxabicyclo-[2.2.2]-octane.
   1.14 Esters of β-(3,5-dicyclohexyl-4-hydroxyphenyl)propionic acid with mono- or polyhydric alcohols, e.g. with methanol, ethanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl) isocyanurate, N,N′-bis(hydroxyethyl)oxalic acid diamide, 3-thiaundecanol, 3-thiapentadecanol, trimethylhexanediol, trimethylolpropane, 4-hydroxymethyl-1-phospha-2,6,7-trioxabicyclo-[2.2.2]-octane.
   1.15 Esters of 3,5-di-tert.-butyl-4-hydroxyphenyl acetic acid with mono- or polyhydric alcohols, e.g. with methanol, ethanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl) isocyanurate, N,N′-bis(hydroxyethyl)oxalic acid diamide, 3-thiaundecanol, 3-thiapentadecanol, trimethylhexanediol, trimethylolpropane, 4-hydroxymethyl-1-phospha-2,6,7-trioxabicyclo-[2.2.2]-octane.
   1.16. Amides of β-(3,5-di-tert-butyl-4-hydroxyphenyl)propionic acid e.g. N,N′-bis(3,5-di-tert-butyl-4-hydroxyphenylpropionyl)hexamethylene-diamine, N,N′-bis(3,5-di-tert-butyl-4-hydroxyphenylpropionyl)tri-methylene-diamine, N,N′-bis(3,5-di-tert-butyl-4-hydroxyphenylpropionyl)-hydrazine.
2. UV absorbers and light stabilisers
   2.1. 2-(2′-Hydroxyphenyl)benzotriazoles, for example the 5′-methyl, 3′,5′-di-tert-butyl, 5′-tert-butyl, 5′-(1,1,3,3-tetramethylbutyl), 5-chloro-3′,5′-di-tert-butyl, 5-chloro-3′-tert-butyl-5′-methyl, 3′-sec-butyl-5′-tert-butyl, 4′-octoxy, 3′,5′-di-tert-amyl and 3′,5′-bis(α,α-dimethylbenzyl), mixture of 5-chloro-3′-tert.-butyl-5′-(2-octyloxycarbonylethyl)- and 5-chloro-3′-tert.-butyl-5′-[2-(2-ethylhexyloxy)-carbonylethyl]-, 5-chloro-3′-tert.-butyl-5′-(2-methoxycarbonylethyl)-, 3′-tert.-butyl-5′-(2-methoxycarbonylethyl)-, 3′-tert.-butyl-5′-(2-octyloxycarbonylethyl)-, 3′-tert.-butyl-5′-[2-(2-ethylhexyloxy)-carbonylethyl]-, 3′-dodecyl-5′-methyl- and 3′-tert.-butyl-5′-(2-isooctyloxycarbonylethyl)-2′-hydroxyphenyl-2H-benztriazole(2), 2,2′-methylene-bis[4-(1,1,3,3-tetramethylbutyl)-6-benztriazole-2-yl-phenol]; product of ester interchange of 2-[3′-tert.-butyl-5′-(2-methoxycarbonylethyl)-2′-hydroxy-phenyl]-2H-benztriazole with polyethylene glycol 300; [R-CH₂CH₂-COO(CH₂)₃⁆₂ with R=3′-tert.-butyl-4′-hydroxy-5′-2H-benzotriazole-2-yl-phenyl.
   2.2. 2-Hydroxybenzophenones, for example the 4-hydroxy, 4-methoxy, 4-octoxy, 4-decyloxy, 4-dodecyloxy, 4-benzyloxy, 4,2′,4′-trihydroxy and 2′-hydroxy-4,4′-dimethoxy derivatives.
   2.3. Esters of substituted and unsubstituted benzoic acids, as for example 4-tert.butylphenyl salicylate, phenyl salicylate, octylphenyl salicylate, dibenzoylresorcinol, bis-(4-tert.butylbenzoyl)-resorcinol, benzoylresorcinol, 2,4-di-tert.butylphenyl 3,5-di-tert.butyl-4-hydroxybenzoate, hexadecyl 3,5-di-tert.butyl-4-hydroxybenzoate, octadecyl 3,5-di-tert.-butyl-4-hydroxybenzoate, 2 methyl-4,6-di-tert.-butylphenyl 3,5-di-tert.-butyl-4-hydroxybenzoate.
   2.4. Acrylates, for example ethyl α-cyano-β,β-diphenylacrylate, isooctyl α-cyano-β,β-diphenylacrylate, methyl α-carbomethoxycinnamate, methyl α-cyano-β-methyl-p-methoxy-cinnamate, butyl α-cyano-β-methyl-p-methoxy-cinnamate, methyl α-carbomethoxy-p-methoxycinnamate and N-(β-carbomethoxy-β-cyanovinyl)-2-methylindoline.
   2.5. Nickel compounds, for example nickel complexes of 2,2′-thio-bis-[4-(1,1,3,3-tetramethylbutyl)phenol], such as the 1:1 or 1:2 complex, with or without additional ligands such as n-butylamine, triethanolamine or N-cyclohexyldiethanolamine, nickel dibutyldithiocarbamate, nickel salts of 4-hydroxy-3,5-di-tert-butylbenzyl-phosphonic acid monoalkyl esters, e.g. of the methyl or ethyl ester, nickel complexes of ketoximes, e.g. of 2-hydroxy-4-methyl-phenyl undecyl ketoxime, nickel complexes of 1-phenyl-4-lauroyl-5-hydroxypyrazole, with or without additional ligands.
   2.6. Sterically hindered amines, for example bis(2,2,6,6-tetramethyl-piperidyl) sebacate, bis-(2,2,6,6-tetramethyl-piperidyl) succinate, bis(1,2,2,6,6-pentamethylpiperidyl) sebacate, bis(1,2,2,6,6-pentamethylpiperidyl) n-butyl-3,5-di-tert-butyl-4-hydroxy-benzylmalonate, the condensation product of 1-(2-hydroxyethyl)-2,2,6,6-tetramethyl-4-hydroxypiperidine and succinic acid, the condensation product of N,N′-bis(2,2,6,6-tetramethyl-4-piperidyl)hexamethylenediamine and 4-tert-octylamino-2,6-dichloro-1,3,5-triazine, tris(2,2,6,6-tetramethyl-4-piperidyl) nitrilotriacetate, tetrakis(2,2,6,6-tetramethyl-4-piperidyl)-1,2,3,4-butane-tetracarboxylate, 1,1′-(1,2-ethanediyl)bis-(3,3,5,5-tetramethyl-piperazinone), 4-benzoyl-2,2,6,6-tetramethylpiperidine, 4-stearyloxy-2,2,6,6-tetramethylpiperidine, bis-(1,2,2,6,6-pentamethylpiperidyl)-2-n-butyl-2-(2-hydroxy-3,5-di-tert.-butylbenzyl) malonate, 3-n-octyl-7,7,9,9-tetramethyl-1,3,8-triazasprio[4.5]decan-2,4-dion, bis-(1-octyloxy-2,2,6,6-tetramethylpiperidyl)sebacate, bis-(1-octyloxy-2,2,6,6-tetramethylpiperidyl) succinate, product of condensation of N,N′-bis-(2,2,6,6-tetramethyl-4-piperidyl)-hexamethylene diamine and 4-morpholino-2,6-dichloro-1,3,5-triazine, product of condensation of-chloro-4,6-di-(4-n-butylamino-2,2,6,6-tetramethylpiperidyl)-1,3,5-triazine and 1,2-bis-(3-aminopropylamino)ethane, product of condensation of 2-chloro-4,6-di-(4-n-butylamino-1,2,2,6,6-pentamethylpiperidyl)-1,3,5-triazine and 1,2-bis-(3-aminopropylamino)ethane, 8-acetyl-3-dodecyl-7,7,9,9-tetramethyl-1,3,8-triazaspiro[4.5]decane-2,4-dion, 3-dodecyl-1-(2,2,6,6-tetramethyl-4-piperidyl)pyrrolidin-2,5-dion, 3-dodecyl-1-(1,2,2,6,6-pentamethyl-4-piperidyl)-pyrrolidin-2,5-dion.
   2.7. Oxalic acid diamides, for example 4,4′-dioctyloxyoxanilide, 2,2′-dioctyloxy-5,5′-di-tert-butyloxanilide, 2,2′-didodecyloxy-5,5′-di-tert-butyloxanilide, 2-ethoxy-2′-ethyloxanilide, N,N′-bis(3-dimethylamino-propyl)oxalamide, 2-ethoxy-5-tert-butyl-2′-ethyloxanilide and its mixture with 2-ethoxy-2′-ethyl-5,4′-di-tert-butyloxanilide and mixtures of ortho- and para-methoxy-disubstituted oxanilides and mixtures of o- and p-ethoxy-disubstituted oxanilides.
   2.8. 2-(2-Hydroxyphenyl)-1,3,5-triazines, for example 2,4,6-tris(2-hydroxy-4-octyloxy-phenyl)-1,3,5-triazine, 2-(2-hydroxy-4-octyloxy-phenyl)-4,6-bis(2,4-dimethylphenyl)-1,3,5-triazine, 2-(2,4-dihydroxy-phenyl)-4,6-bis(2,4-dimethylphenyl)-1,3,5-triazine, 2,4-bis(2-hydroxy-4-propyloxyphenyl)-6-(2,4-dimethylphenyl)-1,3,5-triazine, 2-(2-hydroxy-4-octyloxyphenyl)-4,6-bis(4-methylphenyl)-1,3,5-triazine, 2-(2-hydroxy-4-dodecyloxyphenyl)-4,6-bis(2,4-dimethylphenyl)-1,3,5-triazine, 2-[2-hydroxy-4-(2-hydroxy-3-butyloxy-propyloxy)phenyl]-4,6-bis(2,4-dimethyl)-1,3,5-triazine, 2-[2-hydroxy-4-(2-hydroxy-3-octyloxy-propyloxy)phenyl]-4,6-bis(2,4-dimethyl)-1,3,5-triazine.
3. Metal deactivators, for example N,N′-diphenyloxalic acid diamide, N-salicylal-N′-salicyloylhydrazine, N,N′-bis(salicyloyl)hydrazine, N,N′-bis(3,5-di-tert-butyl-4-hydroxyphenylpropionyl)hydrazine , 3-salicyl-oylamino-1,2,4-triazole, bis(benzylidene) oxalodihydrazide, Oxanilide, isophthalic acid dihydrazide, sebacic acid-bis-phenylhydrazide, N,N′-diacetal-adipinic acid dihydrazide, N,N′-bis-salicyloyl-oxalic acid dihydrazide, N,N′-bis-salicyloyl-thiopropionic acid dihydrazide.
4. Phosphites and phosphonites, for example triphenyl phosphite, diphenyl alkyl phosphites, phenyl dialkyl phosphites, tris(nonylphenyl) phosphite, trilauryl phosphite, trioctadecyl phosphite, distearyl pentaerythritol diphosphite, tris(2,4-di-tert-butylphenyl) phosphite, diiso- decyl pentaerythritol diphosphite, bis(2,4-di-tert.-butylphenyl) pentaerythritol diphosphite, bis-(2,6-di-tert.-butyl-4-methylphenyl)-pentaeryt hritol diphosphite, bis-isodecyloxy-pentaerythritol diphosphite, bis-(2,4-di-tert.-butyl-6-methylphenyl)-pentaerythritol diphosphite, bis-(2,4,6-tri-tert.-butylphenyl)-pentaerythritol diphsophite, tristearyl sorbitol triphosphite, tetrakis(2,4-di-tert-butylphenyl) 4,4′-biphenylene diphosphonite, 6-isooctyloxy-2,4,8,10-tetra-tert.-butyl-12H-dibenz[d,g]-1,3,2-dioxaphosphocin, 6-fluoro-2,4,8,10-tetra-tert.-butyl-12-methyl-dibenz[d,g]-1,3,2-dioxaphosphocin.
5. Peroxide scavengers, for example esters of β-thiodipropionic acid, for example the lauryl, stearyl, myristyl or tridecyl esters, mercaptobenzimidazole or the zinc salt of 2-mercaptobenzimidazole, zinc dibutyldithiocarbamate, dioctadecyl disulfide, pentaerythritol tetrakis(β-dodecyl-mercapto)propionate.
6. Polyamide stabilisers, for example, copper salts in combination with iodides and/or phosphorus compounds and salts of divalent manganese.
7. Basic co-stabilisers, for example, melamine, polyvinylpyrrolidone, dicyandiamide, triallyl cyanurate, urea derivatives, hydrazine derivatives, amines, polyamides, polyurethanes, alkali metal salts and alkaline earth metal salts of higher fatty acids for example Ca stearate, Zn stearate, Mc, behenate, Ma stearate, Na ricinoleate and K palmitate, antimony pyrocatecholate or zinc pyrocatecholate.
8. Nucleating agents, for example, 4-tert.butyl-benzoic acid, adipic acid, diphenylacetic acid.
9. Fillers and reinforcing agents, for example, calcium carbonate, silicates, glass fibres, asbestos, talc, kaolin, mica, barium sulfate, metal oxides and hydroxydes, carbon black, graphite.
10. Other additives, for example, plasticisers, lubricants, emulsifiers, pigments, optical brighteners, flameproofing agents, antistatic agents and blowing agents.

Frequently the stabilized polymer compositions additionally contain from about 0.01 to about 5%, preferably from about 0.025 to about 2%, and especially from about 0.1 to about 1% by weight of various other stabilizers, such as the materials listed above.

As pointed out above, polymer compositions according to the invention preferably contain as other stabilizer a phenolic antioxidant and/or a hindered amine. Some examples for phenolic antioxidants are the compounds listed above under items 1.1., 1.2. and 1.5.. Some examples for hindered amines are the compounds listed above under item 2.6..

The phenolic antioxidant of particular interest is selected from the group consisting of n-octadecyl 3,5-di-tert-butyl-4-hydroxyhydrocinnamate, neopentanetetrayl tetrakis(3,5-di-tert-butyl-4-hydroxyhydrocinammate), di-n-octadecyl 3,5-di-tert-butyl-4-hydroxybenzylphosphonate, 1,3,5-tris(3,5-di-tert-butyl-4-hydroxybenzyl)isocyanurate, thiodiethylene bis(3,5-di-tert-butyl-4-hydroxyhydrocinnamate), 1,3,5-trimethyl-2,4,6-tris-(3,5-di-tert-butyl-4-hydroxybenzyl)benzene, 3,6-dioxaoctamethylene bis(3-methyl-5-tert-butyl-4-hydroxyhydrocinnamate), 2,6-di-tert-butyl-p-cresol, 2,2′-ethylidene-bis(4,6-di-tert-butylphenol), 1,3,5-tris(2,6-dimethyl-4-tert-butyl-3-hydroxybenzyl)isocynurate, 1,1,3,-tris(2-methyl-4-hydroxy-5-tert-butylphenyl)butane, 1,3,5-tris[2-(3,5-di-tert-butyl-4-hydroxyhydrocinnamoyloxy)ethyl]isocyanurate, 3,5-di-(3,5-di-tert-butyl-4-hydroxybenzyl)mesitol, hexamethylene bis(3,5-di-tert-butyl-4-hydroxyhydrocinnamate), 1-(3,5-di-tert-butyl-4-hydroxyanilino)-3,5-di(octylthio)-s-triazine, N,N′-hexamethylene-bis(3,5-di-tert-butyl-4-hydroxyhydrocinnamamide), calcium bis(ethyl3,5-di-tert-butyl-4-hydroxybenzylphosphonate), ethylene bis[3,3-di(3-tert-butyl-4-hydroxyphenyl)butyrate], octyl 3,5-di-tert-butyl-4-hydroxybenzylmercaptoacetate, bis(3,5-di-tert-butyl-4-hydroxyhydrocinnamoyl)hydrazide, and N,N′-bis[2-(3,5-di-tert-butyl-4-hydroxyhydrocinnamoyloxy)-ethyl]-oxamide.

A most preferred phenolic antioxidant is neopentanetetrayl tetrakis(3,5-di-tert-butyl-4-hydroxyhydrocinnamate), n-octadecyl 3,5-di-tert-butyl-4-hydroxyhydrocinnamate, 1,3,5-trimethyl-2,4,6-tris(3,5-di-tert-butyl-4-hydroxybenzyl)benzene, 1,3,5-tris(3,5-di-tert-butyl-4-hydroxybenzyl)isocyanurate, 2,6-di-tert-butyl-p-cresol or 2,2′-ethylidene-bis(4,6-di-tert-butylphenol).

The hindered amine compound of particular interest is selected from the group consisting of bis(2,2,6,6-tetramethylpiperidin-4-yl) sebacate, bis(1,2,2,6,6-pentamethylpiperidin-4-yl) sebacate, di(1,2,2,6,6-pentamethylpiperidin-4-yl) (3,5-di-tert-butyl-4-hydroxybenzyl)butylmalonate, 4-benzoyl-2,2,6,6-tetramethylpiperidine, 4-stearyloxy-2,2,6,6-tetramethylpiperidine, 3-n-octyl-7,7,9,9-tetramethyl-1,3,8-triaza-spiro[4.5]decane-2,4-dione, tris(2,2,6,6-tetramethylpiperidin-4-yl) nitrilotriacetate, 1,2-bis(2,2,6,6-tetramethyl-3-oxopiperazin-4-yl)ethane, 2,2,4,4-tetramethyl-7-oxa-3,20-diaza-21-oxodispiro[5.1.11.2]heneicosane, polycondensation product of 2,4-dichloro-6-tert-octylamino-s-triazine and 4,4′-hexamethylenebis(amino-2,2,6,6-tetramethyl_piperidine), polycondensation product of 1-(2-hydroxyethyl)-2,2,6,6-tetramethyl-4-hydroxypiperidine and succinic acid, polycondensation product of 4,4′-hexamethylenebis-(amino-2,2,6,6-tetramethylpiperidine) and 1,2-dibromoethane, tetrakis(2,2,6,6-tetramethyl_piperidin-4-yl)1,2,3,4-butanetetracarboxylate, tetrakis(1,2,2,6,6-pentamethylpiperidin-4-yl)1,2,3,4-butanetetracarboxylate, polycondensation product of 2,4-dichloro-6-morpholino-s-triazine and 4,4′-hexamethylenebis(amino-2,2,6,6-tetramethylpiperidine), N,N′,N˝,N‴-tetrakis[(4,6-bis(butyl-2,2,6,6-tetramethyl-piperidin-4-yl)-amino-s-triazin-2-yl]-1,10-diamino-4,7-diazadecane, mixed [2,2,6,6-tetramethylpiperidin-4-yl/β,β,β′,β′-tetramethyl-3,9-(2,4,8,10-tetraoxaspiro[5.5]-undecane)diethyl]1,2,3,4-butanetetracarboxylate, mixed [1,2,2,6,6-pentamethylpiperidin-4-yl/β,β,β′,β′-tetramethyl-3,9-(2,4,8,10-tetraoxaspiro[5.5]undecane)diethyl] 1,2,3,4-butanetetracarboxylate, octamethylene bis(2,2,6,6-tetramethylpiperidin-4-carboxylate) 4,4′-ethylenebis(2,2,6,6-tetramethylpiperazin-3-one) and bis(1-octyloxy-2,2,6,6-tetramethylpiperidin-4-yl) sebacate.

A most preferred hindered amine compound is bis(2,2,6,6-tetramethylpiperidin-4-yl sebacate, the polycondensation product of 1-(2-hydroxyethyl)-2,2,6,6-tetramethyl-4-hydroxypiperidine and succinic acid, the polycondensation product of 2,4-dichloro-6-tert-octylamino-2-triazine and 4,4′-hexamethylenebis(amino-2,2,6,6-tetramethylpiperidine), N,N′,N˝,N‴-tetrakis(4,6-bis(butyl(2,2,6,6-tetramethyl-piperidin-4-yl)amino)-s-triazine-2-yl]-1,10-diamino-4,7-diazadecane or bis(1-octyloxy-2,2,6,6-tetramethylpiperidin-4-yl) sebacate.

The following examples are presented for the purpose of illustration only and are not to be construed to limit the nature or scope of the instant invention in any manner whatsoever. Percentages given are by weight unless otherwise indicated.

### Example 1

### 2,6-Diphenylpiperidin-4-yl Laurate

To a -70 °C solution of 2.50 g, (9.9 mmol) of 2,6-diphenyl-4-hydroxypiperidine in 80 ml of dry tetrahydrofuran (THF), 4.2 mL (10.5 mmol) of n-butyllithium (2.5 M in hexanes) is added dropwise. The mixture is warmed to -20°C and stirred for 30 minutes. The solution is then cooled again to -70°C and a solution of 2.2 g (9.9 mmol) of lauroyl chloride in 20 mL of dry THF is then added dropwise. The mixture is warmed to room temperature and stirred for 18 hours. The mixture is added to 250 mL of brine and then extracted with 2 x 200 mL of ether. The combined organic layers are dried over anhydrous magnesium sulfate and concentrated under reduced pressure. The residue is purified by liquid chromatography (LC) (silica gel, ethyl acetate/hexane) to give 3.2 g (74%) of the title compound as a white solid: mp 40-42°C.

| | | | | |
|---|---|---|---|---|
| Analysis: | Calcd for C₂₉H₄₁NO₂: | C, 79.9; | H, 9.5; | N, 3.2. |
| | Found: | C, 79.9; | H, 9.9; | N, 3.6. |

### Example 2

### 1-Hydroxy-2,6-diphenylpiperidin-4-yl Laurate

A solution of 590 mL (27.7 mmol) of dimethyldioxirane, 0.047 M in acetone (prepared by the procedure of Eaton and Wicks, J. Org. Chem. 1988, 53, 5353) is added via a cannula to a 0°C solution of 12.1 g (27.7 mmol) of 2,6-diphenyl-4-piperidyl laurate in 200 mL of acetone. After stirring for 10 minutes at 0°C, the reaction mixture is concentrated under reduced pressure and the residue is recrystallized from methanol to yield 9.7 g (78% yield) of the title compound as a white solid: mp 89-90°C.

| | | | | |
|---|---|---|---|---|
| Analysis: | Calcd for C₂₉H₄₁NO₂: | C, 77. 1; | H, 9.2; | N, 3.1. |
| | Found: | C, 77. 1; | H, 9.2; | N, 2.9. |

### Example 3

### Bis-(2,6-diphenylpiperidin-4-yl) Sebacate

The general procedure of Example 1 is repeated using 2.9 g (11.5 mmol) of 2,6-diphenyl-4-hydroxypiperidine, 4.9 mL (12.0 mmol) of n-butyllithium (2.5 M in hexanes) and 1.35 g (5.6 mmol) of sebacoyl chloride. 3.5 g (93% yield) of the title compound is isolated.

### Example 4

### Bis(1-hydroxy-2,6-diphenylpiperidin-4-yl) Sebacate

The general procedure of Example 2 is repeated using 2.0 g (3.0 mmol) of bis-(2,6-diphenylpiperidin-4-yl) sebacate and 82.8 mL (6.0 mmol) of dimethyldioxirane (0.072M in acetone). 1.6 g (76% yield) of the title compound is isolated after recrystallization from methanol: mp 138-158°C.

| | | | | |
|---|---|---|---|---|
| Analysis: | Calcd for C₄₄H₅₂N₂O₆: | C, 75.0; | H, 7.4; | N, 4.0. |
| | Found: | C, 74.5; | H, 7.4; | N, 3.8. |

### Example 5

### 2,6-Diphenyl-3-methylpiperidin-4-yl Laurate

The general procedure of Example 1 is repeated using 22.8 g (85.2 mmol) of 2,6-diphenyl-3-methyl-4-hydroxypiperidine, 34 mL (85 mmol) of n-butyllithium (2.5 M in hexanes) and 18.6 g (85.2 mmol) of lauroyl chloride. 25.0 g (65% yield) of the title compound is isolated after purification by LC (silica gel, ethyl acetate/hexane): mp 56-59°C.

| | | | | |
|---|---|---|---|---|
| Analysis: | Calcd for C₃₀H₄₃NO₂: | C, 80.1; | H, 9.6; | N, 3.1. |
| | Found: | C, 80.8; | H, 10.1; | N, 3.0. |

### Example 6

### 1-Hydroxy-2,6-diphenyl-3-methylpiperidin-4-yl Laurate

The general procedure of Example 2 is repeated using 11.5 g (25.5 mmol) of 2,6-diphenyl-3-methylpiperidin-4-yl laurate and 570 mL (25.5 mmol) of dimethyldioxirane (0.045 M in acetone). 11.8 g (99% yield) of the title compound is isolated: mp 84-91°C.

| | | | | |
|---|---|---|---|---|
| Analysis: | Calcd for C₃₀H₄₃NO₃: | C, 77.4; | H, 9.3; | N, 3.0. |
| | Found: | C, 77.0; | H, 9.2; | N, 2.8. |

### Example 7

### 2,6-Diphenyl-3,5-dimethylpiperidin-4-yl Laurate

The general procedure of Example 1 is repeated using 28.1 g (0.10 mol) of 2,6-diphenyl-3,5-dimethyl-4-hydroxypiperidine, 40 mL (0.10 mol) of n-butyllithium (2.5M in hexanes) and 21.8 g (0.10 mol) of lauroyl chloride. 34.5 g (74% yield) of the title compound is isolated after purification by LC (silica gel, ethyl acetate/hexane): mp 74-76°C.

| | | | | |
|---|---|---|---|---|
| Analysis: | Calcd for C₃₁H₄₅NO₂: | C, 80.3; | H, 9.8; | N, 3.0. |
| | Found: | C, 80.0; | H,10.2; | N, 2.6. |

### Example 8

### 1-Hydroxy-2,6-diphenyl-3,5-dimethylpiperidin-4-yl Laurate

The general procedure of Example 2 is repeated using 29.9 g (64.5 mmol) of 2,6-diphenyl-3,5-dimethylpiperidin-4-yl laurate and 1.24 L (64.5 mmol) of dimethyldioxirane (0.050 M in acetone). 30.8 g (98% yield) of the title compound is isolated: mp 108-112°C.

| | | | | |
|---|---|---|---|---|
| Analysis: | Calcd for C₃₁H₄₅NO₃: | C, 77.6; | H, 9.5; | N, 2.9. |
| | Found: | C, 77.5; | H, 9.8; | N, 2.5. |

### Example 9

### Bis-(2,6-diphenyl-3,5-dimethylpiperidin-4-yl) Sebacate

The general procedure of Example 1 is repeated using 18.0 g (64 mmol) of 2,6-diphenyl-3,5-dimethyl-4-hydroxypiperidine, 25.5 mL (64 mmol) of n-butyllithium (2.5M in hexanes) and 7.65g (32 mmol) of sebacoyl chloride. 2.7 g (12% yield) of the title compound is isolated after purification by LC (silica gel, ethyl acetate/hexane: mp 128-135°C.

| | | | | |
|---|---|---|---|---|
| Analysis: | Calcd for C₄₈H₆₀N₂O₄: | C, 79.1; | H, 8.3; | N, 3.8. |
| | Found: | C, 78.6; | H, 8.2; | N, 3.7. |

### Example 10

### Bis-(1-hydroxy-2,6-diphenyl-3,5-dimethylpiperidin-4-yl) Sebacate

The general procedure of Example 2 is repeated using 2.09 g (2.86 mmol) of bis-(2,6-diphenyl-3,5-dimethylpiperidin-4-yl) sebacate and 82 mL (5.72 mmol) of dimethyldioxirane (0.070 M in acetone). 2.18 g (100% yield) of the title compound is isolated: mp 190-191°C.

| | | | | |
|---|---|---|---|---|
| Analysis: | Calcd for C₄₈H₆₀N₂O₆: | C, 75.8; | H, 8.0; | N, 3.7. |
| | Found: | C, 75.4; | H, 8.0; | N, 3.6. |

### Example 11

### N-(2,6-Diphenyl-3,5-dimethylpiperidin-4-yl)lauramide

To a stirred 0°C solution of 12.7 g (45.3 mmol) of 2,6-diphenyl-3,5-dimethyl-4-aminopiperidine and 5.1 g (50 mmol) of triethylamine in 150 ml of methylene chloride is added dropwise a solution of 9.9 g (45.3 mmol) of lauroyl chloride. After stirring for 30 minutes at 0°C, the reaction mixture is washed with water (2 x 200 ml) followed by a saturated aqueous sodium bicarbonate solution (300 ml). The organic phase is separated, dried over anhydrous magnesium sulfate and then concentrated under reduced pressure. The resulting solid is recrystallized from ethanol to give 15.4 g (73 % yield) of the title compound as a white solid melting at 112-114°C.

| | | | | |
|---|---|---|---|---|
| Analysis: | Calcd for C₃₁H₄₆N₂O: | C, 80.5; | H, 10.0; | N, 6.0. |
| | Found | C, 80.5; | H, 10.4; | N, 6.0. |

### Example 12

### N-(1-Hydroxy-2,6-diphenyl-3,5-dimethylpiperidin-4-yl)lauramide

Following the general procedure of Example 2 and using 8.56 g (18.5 mmol) of N-(2,6-diphenyl-3,5-dimethylpiperidin-4-yl)lauramide, prepared in Example 11, and 462 ml of a 0.04 molar solution of dimethyldioxirane (18.5 mmol) in acetone, the title compound is isolated in a yield of 7.37 g (70 %) after purification by LC (silica gel; ethyl acetate:hexanes) as a white solid melting at 58-62°C.

| | | | | |
|---|---|---|---|---|
| Analysis: | Calcd C₃₁H₄₆N₂O₂: | C, 77.8; | H, 9.7; | N, 5.8. |
| | Found: | C, 77.6; | H, 10.2; | N, 5.7. |

### Example 13

### Process Stabilization of Polypropylene at 525°F (274°C)

This example illustrates the process stabilizing effectiveness of the instant compounds in polypropylene.

The base formuation comprises unstabilized, old technology polypropylene (PROFAX 6501, Himont) containing 0.075% by weight of calcium stearate. The test additives are incorporated into the polyproyplene by dry blending or, when the additive is a liquid, using a minimum amount of methylene chloride solvent. The solvent is then removed by evaporation under reduced pressure. The stabilized resin formulation is extruded at 90 rpm from a 1 inch (2.54 cm) diameter extruder at 525°F (274°C) with a residence time of 90 seconds.

After each of the first and fifth extrusions, the melt flow rate (in grams/10 minutes) is determined by ASTM method D1238 on the pellets obtained from the extruder. The results are given in the table below.

| Additive | Concentration (% by weight) | Melt Flow after Extrusion | |
|---|---|---|---|
| | | 1 | 5 |
| None | - | 10.5 | 61.7 |
| Compound of Example 2 | 0.075 | 4.5 | 7.0 |
| Compound of Example 4 | 0.075 | 4.3 | 8.8 |
| Compound of Example 8 | 0.075 | 4.3 | 8.4 |
| Compound of Example 10 | 0.075 | 4.2 | 7.6 |

These results show that the instant 1-hydroxy-2,6-diaryl-4-acyloxypiperidines provide melt flow stabilization to polypropylene as processing stabilizers.

### Example 14

### Process Stabilization of Polypropylene at 525°F (274°C)

This example illustrates the process stabilizing effectiveness of the instant compounds in polypropylene in formulations containing a phenolic antioxidant.

The results using the procedure described in Example 13 on polypropylene formulations containing an instant compound and a phenolic antioxidant are given in the table below.

| Additive* | Concentration (% by weight) | Melt Flow after Extrusion | |
|---|---|---|---|
| | | 1 | 5 |
| AO A | 0.075 | 6.7 | 20.2 |
| AO A plus Example 2 | 0.075 | 3.8 | 6.5 |
| | 0.075 | | |
| AO A plus Example 4 | 0.075 | 3.9 | 8.6 |
| | 0.075 | | |
| AO A plus Example 8 | 0.075 | 4.3 | 7.4 |
| | 0.075 | | |
| AO A Plus Example 10 | 0.075 | 4.2 | 8.0 |
| | 0.075 | | |

| | | | |
|---|---|---|---|
| *AO A is neopentanetetrayl tetrakis(3,5-di-tert-butyl-4-hydroxyhydrocinnamate). | | | |

These results show that the instant 1-hydroxy-2,6-diaryl-4-acyloxypiperidines in combination with a phenolic antioxidant provide melt flow stabilization to polypropylene as processing stabilizers.

### Example 15

### Color Stabilization of Polypropylene

This example illustrates the color stabilizing effectiveness of the instant compounds in combination with a phenolic antioxidant in polypropylene.

Using the procedure described in Example 13, polypropylene containing a phenolic antioxidant in combination with an instant compound is extruded into pellets. Using the pellets obtained after each of the first and fifth extrusions as described in Example 13, the pellets are compression molded into 125 mil (3.2 mm) thick plaques at 193°C. Specimen yellowness index (YI) values are determinated according to ASTM method D 1925. Lower YI values indicate less discoloration. The results are given in the table below.

| Additive* | Concentration (% by weight) | Yellowness YI after Extrusion | |
|---|---|---|---|
| | | 1 | 5 |
| AO A | 0.075 | 8.05 | 8.60 |
| AO A plus Example 2 | 0.075 | 6.45 | 7.40 |
| | 0.075 | | |
| AO A plus Example 4 | 0.075 | 6.70 | 7.69 |
| | 0.075 | | |
| AO A plus Example 8 | 0.075 | 5.45 | 6.34 |
| | 0.075 | | |
| AO A plus Example 10 | 0.075 | 5.91 | 6.48 |
| | 0.075 | | |

| | | | |
|---|---|---|---|
| *AO A is neopentanetetrayl tetrakis(3,5-di-tert-butyl-4-hydroxyhydrocinnamate). | | | |

These results show that the instant 1-hydroxy-2,6-diaryl-4-acyloxypiperidines in combination with a phenolic antioxidant provide color stabilization to polypropylene as processing stabilizers.

### Example 16

### Long Term Heat Aging Stability of Polypropylene

Extruded pellets (of Example 14), after the first pass, are compression molded into 125 mil (3.2 mm) plaques at 450°F (232°C) and then oven aged at 150°C in a forced draft oven equipped with a rotating carousel. The time, in days, to reach a yellowness index (YI) color of 50 units is deemed to represent failure. The results are given in the table below.

| Additive* | Concentration (% by weight) | Days to Failure |
|---|---|---|
| AO A | 0.075 | 43 |
| AO A plus Example 2 | 0.075 | 50 |
| | 0.075 | |

| | | |
|---|---|---|
| *AO A is neopentanetetrayl tetrakis(3,5-di-tert-butyl-4-hydroxyhydrocinnamate). | | |

### Example 17

### Long Term Heat Aging Stability of Polypropylene

Extruded pellets (of Example 14), after the first pass, are compression molded into 40 mil (1 mm) plaques at 450°F (232°C) and then oven aged at 150°C in a forced draft oven equipped with a rotating carousel. The time, in days, to physical failure is determined by a 90° bend test. The results are given in the table below.

| Additive* | Concentration (% by weight) | Days to Failure |
|---|---|---|
| AO A | 0.075 | 28 |
| AO A plus Example 2 | 0.075 | 31 |
| | 0.075 | |
| AO A plus Example 4 | 0.075 | 38 |
| | 0.075 | |
| AO A plus Example 8 | 0.075 | 38 |
| | 0.075 | |
| AO A plus Example 10 | 0.075 | 34 |
| | 0.075 | |

| | | |
|---|---|---|
| *AO A is neopentanetetrayl tetrakis(3,5-di-tert-butyl-4-hydroxyhydrocinnamate). | | |

### Example 18

### Oxidative Induction Time

Extruded pellets (of Example 14), after the first pass, are analyzed by DSC using aluminum pans/isothermal at 190°C under oxygen according to ASTM-3895-80. The time of onset and peak are is given in minutes. The results are given in the table below.

| Additive* | Concentration (% by weight) | OIT Onset Minutes | OIT Peak Minutes |
|---|---|---|---|
| AO A | 0.075 | 16 | 21 |
| AO A plus Example 2 | 0.075 | 42 | 50 |
| | 0.075 | | |
| AO A plus Example 4 | 0.075 | 31 | 38 |
| | 0.075 | | |
| AO A plus Example 8 | 0.075 | 30 | 38 |
| | 0.075 | | |
| AO A plus Example 10 | 0.075 | 28 | 37 |
| | 0.075 | | |

| | | | |
|---|---|---|---|
| *AO A is neopentanetetrayl tetrakis(3,5-di-tert-butyl-4-hydroxyhydrocinnamate). | | | |

## Claims

1. A compound which is a substituted 1-hydroxy-2,6-diaryl-4-acyloxypiperidine or 1-hydroxy-2,6-diaryl-4-acylaminopiperidine of formula I 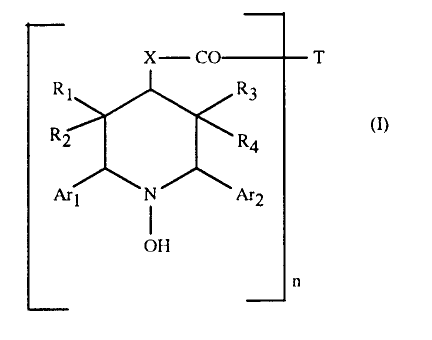 wherein
n is 1, 2, 3 or 4;
X is -O- or -NE-,
E is hydrogen, alkyl of 1 to 20 carbons or cycloalkyl of 5 to 12 carbon atoms,
Ar₁ and Ar₂ are independently aryl of 6 to 10 carbon atoms; or said aryl substituted by one to three substituents selected from the group consisting of alkyl of 1 to 20 carbon atoms; cycloalkyl of 5 to 12 carbon atoms; phenylalkyl of 7 to 15 carbon atoms; -COOR₅ where R₅ is hydrogen or alkyl of 1 to 20 carbons; -COR₆ where R₆ is alkyl of 1 to 20 carbons; -NR₇R₈ where R₇ and R₈ are independently hydrogen or alkyl of 1 to 20 carbons; -SR₉ where R₉ is aryl of 6 to 10 carbon atoms or alkyl of 1 to 20 carbon atoms; -OH; -OCH₃; -CN; -CF₃; -NO₂; -F; -Cl; -Br and -I;
R₁, R₂, R₃and R₄ are independently hydrogen; a linear or branched alkyl of 1 to 30 carbon atoms; or said alkyl terminated with -OR₁₀, -NR₁₁R₁₂, -SR₁₃, -COOR₁₄ or -CONR₁₅R₁₆, where R₁₀, R₁₁, R₁₂, R₁₃ and R₁₄ are independently alkyl of 1 to 20 carbon atoms or alkenyl of 3 to 18 carbon atoms, and R₁₅ and R₁₆ are independently hydrogen or the same meaning as R₁₀; or said alkyl interrupted by one or more -O-, -S-, -SO-, -SO₂-, -CO-, -COO-, -OCO-, -CONR₁₇-, -NR₁₇CO- or -NR₁₈- where R₁₇ and R₁₈ have the same meaning as R₁₅; alkenyl of 3 to 20 carbon atoms; aryl of 6 to 10 carbon atoms; or said aryl substituted by one to three substituents selected from the group consisting of alkyl of 1 to 20 carbon atoms, cycloalkyl of 5 to 12 carbon atoms, and phenylalkyl of 7 to 15 carbon atoms; and
when n is 1, T is alkyl of 1 to 20 carbon atoms or said alkyl interrupted by one or more of -O-, -S-, -SO-, -SO₂-, -CO-, -COO-, -OCO-, -CONR₁₉-,-NR₁₉CO- or -NR₂₀- where R₁₉ and R₂₀ have the same meaning as R₁₅; or aryl or substituted aryl having the same definition as Ar₁;
when n is 2, T is a direct bond; alkylene of 1 to 12 carbon atoms, or said alkylene interrupted by one or more of -O-, -S-, -SO-, -SO₂-, -CO-, -COO-, -OCO-, -CONR₂₁, -NR₂₁CO- or -NR₂₂- where R₂₁ and R₂₂ have the same meaning as R₁₅;
when n is 3, T is alkanetriyl of 3 to 8 carbon atoms; and
when n is 4, T is alkanetetrayl of 4 to 10 carbon atoms.

2. A compound of formula I according to claim 1 wherein
E is hydrogen or alkyl of 1 to 20 carbon atoms;
Ar₁ and Ar₂ are independently phenyl; or phenyl substituted by one to three substituents selected from the group consisting of alkyl of 1 to 12 carbon atoms; cycloalkyl of 5 to 7 carbon atoms; -COOH; -COO-CH₃; -COO-C₂H₅; -CO-CH₃; -CO-C₂H₅; -NH₂; -OH; and -OCH₃;
R₁, R₂, R₃ and R₄ are independently hydrogen, methyl or ethyl; and
when n is 1, T is alkyl of 1 to 20 carbon atoms or said alkyl interrupted by one or more of -O-, -S-, -SO-, -SO₂-, -CO-, -COO-, -OCO- or phenylene;
when n is 2, T is a direct bond; alkylene of 1 to 12 carbon atoms, or said alkylene interrupted by one or more of -O-, -S-, -SO-, -SO₂-, -CO-, -COO- or -OCO-;
when n is 3, T is alkanetriyl of 3 to 8 carbon atoms; and
when n is 4, T is alkanetetrayl of 4 to 10 carbon atoms.

3. A compound according to claim 2 wherein Ar₁ and Ar₂ are the same and each is phenyl or phenyl substituted by methyl.

4. A compound according to claim 2 wherein n is 1 or 2.

5. A compound according to claim 2 wherein each of R₁, R₂, R₃ and R₄ is hydrogen; or R₁ is methyl, and R₂, R₃ and R₄ are hydrogen; or R₁ and R₃ are methyl, and R₂ and R₄ are hydrogen.

6. A compound according to claim 2 wherein n is 1 or 2; E is hydrogen; each of Ar₁ and Ar₂ are phenyl;
each of R₁, R₂, R₃ and R₄ is hydrogen; or R₁ is methyl, and R₂, R₃ and R₄ are hydrogen;
or R₁ and R₃ are methyl, and R₂ and R₄ are hydrogen; and,
when n is 1, T is alkyl of 7 to 17 carbon atoms; and,
when n is 2, T is alkylene of 2 to 10 carbon atoms.

7. A compound according to claim 6 wherein X is -O-.

8. A compound which is a substituted 2,6-diaryl-4-acyloxypiperidine or 2,6-diaryl-4-acylaminopiperidine of formula II 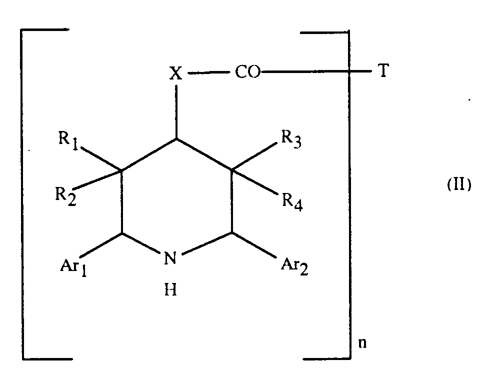 wherein n is 1, 2, 3 or 4;
X is -O- or -NE-,
E is hydrogen, alkyl of 1 to 20 carbon atoms or cycloalkyl of 5 to 12 carbon atoms,
Ar₁ and Ar₂ are independently aryl of 6 to 10 carbon atoms; or said aryl substituted by one to three substituents selected from the group consisting of alkyl of 1 to 20 carbon atoms; cycloalkyl of 5 to 12 carbon atoms; phenylalkyl of 7 to 15 carbon atoms; -COOR₅ where R₅ is hydrogen or alkyl of 1 to 20 carbons; -COR₆ where R₆ is alkyl of 1 to 20 carbons; -NR₇R₈ where R₇ and R₈ are independently hydrogen or alkyl of 1 to 20 carbons; -SR₉ where R₉ is aryl of 6 to 10 carbon atoms or alkyl of 1 to 20 carbon atoms; -OH; -OCH₃; -CN; -CF₃; -NO₂; -F; -Cl; -Br and -I;
R₁, R₂, R₃and R₄ are independently hydrogen; a linear or branched alkyl of 1 to 30 carbon atoms; or said alkyl terminated with -OR₁₀, -NR₁₁R₁₂, -SR₁₃, -COOR₁₄ or -CONR₁₅R₁₆, where R₁₀, R₁₁, R₁₂, R₁₃ and R₁₄ are independently alkyl of 1 to 20 carbon atoms or alkenyl of 3 to 18 carbon atoms, and R₁₅ and R₁₆ are independently hydrogen or the same meaning as R₁₀; or said alkyl interrupted by one or more -O-, -S-, -SO-, -SO₂-, -CO-, -COO-, -OCO-, -CONR₁₇-, -NR₁₇CO- or -NR₁₈- where R₁₇ and R₁₈ have the same meaning as R₁₅; alkenyl of 3 to 20 carbon atoms; aryl of 6 to 10 carbon atoms; or said aryl substituted by one to three substituents selected from the group consisting of alkyl of 1 to 20 carbon atoms, cycloalkyl of 5 to 12 carbon atoms, and phenylalkyl of 7 to 15 carbon atoms; and
when n is 1, T is alkyl of 1 to 20 carbon atoms or said alkyl interrupted by one or more of -O-, -S-, -SO-, -SO₂-, -CO-, -COO-, -OCO-, -CONR₁₉-,-NR₁₉CO- or -NR₂₀- where R₁₉ and R₂₀ have the same meaning as R₁₅; or aryl or substituted aryl having the same definition as Ar₁;
when n is 2, T is a direct bond; alkylene of 1 to 12 carbon atoms, or said alkylene interrupted by one or more of -O-, -S-, -SO-, -SO₂-, -CO-, -COO-, -OCO-, -CONR₂₁, -NR₂₁CO- or -NR₂₂- where R₂₁, and R₂₂ have the same meaning as R₁₅;
when n is 3, T is alkanetriyl of 3 to 8 carbon atoms; and
when n is 4, T is alkanetetrayl of 4 to 10 carbon atoms.

9. A composition which comprises
(a) an organic material subject to oxidative, thermal or actinic degradation, and
(b) an effective stabilizing amount of a compound of formula I as claimed in claim 1.

10. A composition according to claim 9 wherein the organic material is a synthetic polymer.

11. A composition according to claim 10 wherein the synthetic polymer is a polyolefin.

12. A composition according to claim 11 wherein the polyolefin is polyethylene or polypropylene.

13. A composition according to claim 9 which additionally contains an effective stabilizing amount of a phenolic antioxidant and/or a hindered amine.

14. A method of stabilizing organic materials against oxidative, thermal or actinic degradation, which comprises incorporating into said materials at least one compound of formula I according to claim 1.

15. Use of a compound of formula I according to claim 1 as stabilizer for organic materials against oxidative, thermal or actinic degradation.

## Patentansprüche

1. Verbindung, die ein substituiertes 1-Hydroxy-2,6-diaryl-4-acyloxypiperidin oder 1-Hydroxy-2,6-diaryl-4-acylaminopiperidin der Formel I ist, 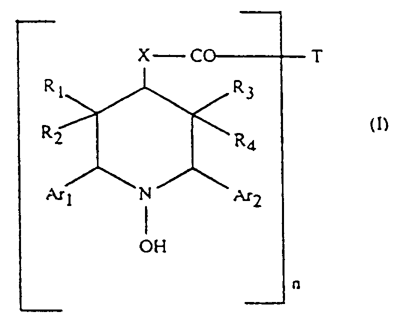 worin n 1, 2, 3 oder 4 ist;
X -O- oder -NE- darstellt;
E Wasserstoff, Alkyl mit 1 bis 20 Kohlenstoffatomen oder Cycloalkyl mit 5 bis 12 Kohlenstoffatomen darstellt;
Ar₁ und Ar₂ unabhängig Aryl mit 6 bis 10 Kohlenstoffatomen; oder das Aryl, substituiert mit ein bis drei Substituenten, ausgewählt aus der Gruppe, bestehend aus Alkyl mit 1 bis 20 Kohlenstoffatomen; Cycloalkyl mit 5 bis 12 Kohlenstoffatomen; Phenylalkyl mit 7 bis 15 Kohlenstoffatomen; -COOR₅, worin R₅ Wasserstoff oder Alkyl mit 1 bis 20 Kohlenstoffatomen bedeutet; -COR₆, worin R₆ Alkyl mit 1 bis 20 Kohlenstoffatomen bedeutet; -NR₇R₈, worin R₇ und R₈ unabhängig Wasserstoff oder Alkyl mit 1 bis 20 Kohlenstoffatomen bedeuten; -SR₉, worin R₉ Aryl mit 6 bis 10 Kohlenstoffatomen oder Alkyl mit 1 bis 20 Kohlenstoffatomen bedeutet; -OH; -OCH₃; -CN; -CF₃; -NO₂; -F; -Cl; -Br und -I; darstellen;
R₁, R₂, R₃ und R₄ unabhängig Wasserstoff, ein lineares oder verzweigtes Alkyl mit 1 bis 30 Kohlenstoffatomen; oder das Alkyl, beendet mit -OR₁₀, -NR₁₁R₁₂, -SR₁₃, -COOR₁₄ oder -CONR₁₅R₁₆, worin R₁₀, R₁₁, R₁₂, R₁₃ und R₁₄ unabhängig Alkyl mit 1 bis 20 Kohlenstoffatomen oder Alkenyl mit 3 bis 18 Kohlenstoffatomen bedeuten, und R₁₅ und R₁₆ unabhängig Wasserstoff oder die gleiche Bedeutung wie R₁₀ aufweisen; oder das Alkyl, unterbrochen durch ein oder mehrere von -O-, -S-, -SO-, -SO₂-, -CO-, -COO-, -OCO-, -CONR₁₇-, -NR₁₇CO- oder -NR₁₈-, worin R₁₇ und R₁₈ die gleichen Bedeutungen wie R₁₅ aufweisen; Alkenyl mit 3 bis 20 Kohlenstoffatomen; Aryl mit 6 bis 10 Kohlenstoffatomen; oder das Aryl, substituiert mit ein bis drei Substituenten, ausgewählt aus der Gruppe, bestehend aus Alkyl mit 1 bis 20 Kohlenstoffatomen, Cycloalkyl mit 5 bis 12 Kohlenstoffatomen und Phenylalkyl mit 7 bis 15 Kohlenstoffatomen, darstellen; und
wenn n 1 ist, T Alkyl mit 1 bis 20 Kohlenstoffatomen oder das Alkyl, unterbrochen durch ein oder mehrere von -O-, -S-, -SO-, -SO₂-, -CO-, -COO-, -OCO-, -CONR₁₉-, -NR₁₉CO- oder -NR₂₀-, worin R₁₉ und R₂₀ die gleiche Bedeutung wie R₁₅ aufweisen; oder Aryl oder substituiertes Aryl mit der gleichen Definition wie Ar₁ darstellt;
wenn n 2 ist, T eine direkte Bindung; Alkylen mit 1 bis 12 Kohlenstoffatomen oder das Alkylen, unterbrochen durch ein oder mehrere von -O-, -S-, -SO-, -SO₂-, -CO-, -COO-, -OCO-, -CONR₂₁, -NR₂₁CO- oder -NR₂₂- bedeutet, worin R₂₁ und R₂₂ die gleiche Bedeutung wie R₁₅ aufweisen;
wenn n 3 ist, T Alkantriyl mit 3 bis 8 Kohlenstoffatomen darstellt; und
wenn n 4 ist, T Alkantetrayl mit 4 bis 10 Kohlenstoffatomen darstellt.

2. Verbindung der Formel I nach Anspruch 1, worin
E Wasserstoff oder Alkyl mit 1 bis 20 Kohlenstoffatomen darstellt;
Ar₁ und Ar₂ unabhängig Phenyl oder Phenyl, substituiert mit ein bis drei Substituenten, ausgewählt aus der Gruppe, bestehend aus Alkyl mit 1 bis 12 Kohlenstoffatomen, Cycloalkyl mit 5 bis 7 Kohlenstoffatomen; -COOH; -COO-CH₃; -COO-C₂H₅; -CO-CH₃; -CO-C₂H₅; -NH₂; -OH und -OCH₃; darstellen;
R₁, R₂, R₃ und R₄ unabhängig Wasserstoff, Methyl oder Ethyl darstellen,
und wenn n 1 ist, T Alkyl mit 1 bis 20 Kohlenstoffatomen oder das Alkyl, unterbrochen durch ein oder mehrere von -O-, -S-, -SO-, -SO₂-, -CO-, -COO-, -OCO- oder Phenylen; darstellt;
wenn n 2 ist, T eine direkte Bindung; Alkylen mit 1 bis 12 Kohlenstoffatomen oder das Alkylen, unterbrochen durch ein oder mehrere von -O-, -S-, -SO-, -SO₂-, -CO-, -COO- oder -OCO-; darstellt;
wenn n 3 ist, T Alkantriyl mit 3 bis 8 Kohlenstoffatomen darstellt; und
wenn n 4 ist, T Alkantetrayl mit 4 bis 10 Kohlenstoffatomen darstellt.

3. Verbindung nach Anspruch 2, worin Ar₁ und Ar₂ gleich sind und jedes Phenyl oder Phenyl, substituiert mit Methyl, darstellt.

4. Verbindung nach Anspruch 2, worin n 1 oder 2 ist.

5. Verbindung nach Anspruch 2, worin jeder der Reste R₁, R₂, R₃ und R₄ Wasserstoff darstellt oder R₁ Methyl darstellt und R₂, R₃ und R₄ Wasserstoff darstellen, oder R₁ und R₃ Methyl darstellen und R₂ und R₄ Wasserstoff darstellen.

6. Verbindung nach Anspruch 2, worin n 1 oder 2 ist; E Wasserstoff darstellt; Ar₁ und Ar₂ jeweils Phenyl darstellen;
jeder der Reste R₁, R₂, R₃ und R₄ Wasserstoff darstellt, oder R₁ Methyl darstellt und R₂, R₃ und R₄ Wasserstoff darstellen; oder R₁ und R₃ Methyl darstellen und R₂ und R₄ Wasserstoff darstellen;
und wenn n 1 ist, T Alkyl mit 7 bis 17 Kohlenstoffatomen darstellt;
und wenn n 2 ist, T Alkylen mit 2 bis 10 Kohlenstoffatomen darstellt.

7. Verbindung nach Anspruch 6, worin X -O- darstellt.

8. Verbindung, nämlich ein substituiertes 2,6-Diaryl-4-acyloxypiperidin oder 2,6-Diaryl-4-acylaminopiperidin der Formel II 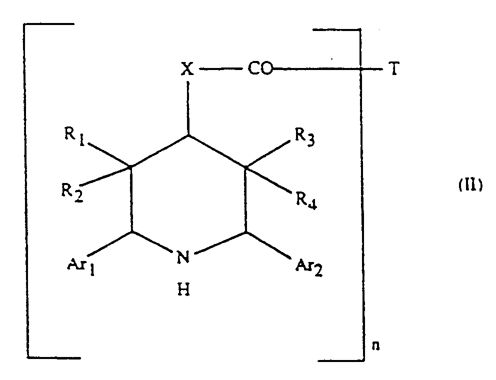 worin n 1, 2, 3 oder 4 ist;
X -O- oder -NE- darstellt;
E Wasserstoff, Alkyl mit 1 bis 20 Kohlenstoffatomen oder Cycloalkyl mit 5 bis 12 Kohlenstoffatomen darstellt; Ar₁ und Ar₂ unabhängig Aryl mit 6 bis 10 Kohlenstoffatomen; oder das Aryl, substituiert mit ein bis drei Substituenten, ausgewählt aus der Gruppe, bestehend aus Alkyl mit 1 bis 20 Kohlenstoffatomen, Cycloalkyl mit 5 bis 12 Kohlenstoffatomen; Phenylalkyl mit 7 bis 15 Kohlenstoffatomen; -COOR₅, worin R₅ Wasserstoff oder Alkyl mit 1 bis 20 Kohlenstoffatomen bedeutet; -COR₆, worin R₆ Alkyl mit 1 bis 20 Kohlenstoffatomen bedeutet; -NR₇R₈, worin R₇ und R₈ unabhängig Wasserstoff oder Alkyl mit 1 bis 20 Kohlenstoffatomen bedeuten; -SR₉, worin R₉ Aryl mit 6 bis 10 Kohlenstoffatomen oder Alkyl mit 1 bis 20 Kohlenstoffatomen bedeutet; -OH; -OCH₃; -CN; -CF₃; -NO₂; -F; -Cl; -Br und -I; darstellen;
R₁, R₂, R₃ und R₄ unabhängig Wasserstoff; ein lineares oder verzweigtes Alkyl mit 1 bis 30 Kohlenstoffatomen oder das Alkyl, beendet mit -OR₁₀, -NR₁₁R₁₂, -SR₁₃, -COOR₁₄ oder -CONR₁₅R₁₆, worin R₁₀, R₁₁, R₁₂, R₁₃ und R₁₄ unabhängig Alkyl mit 1 bis 20 Kohlenstoffatomen oder Alkenyl mit 3 bis 18 Kohlenstoffatomen darstellen und R₁₅ und R₁₆ unabhängig Wasserstoff darstellen oder die gleiche Bedeutung wie R₁₀ aufweisen; oder das Alkyl, unterbrochen durch ein oder mehrere von -O-, -S-, -SO-, -SO₂-, -CO-, -COO-, -OCO-, -CONR₁₇-, -NR₁₇CO- oder -NR₁₈-, worin R₁₇ und R₁₈ die gleiche Bedeutung wie R₁₅ aufweisen; Alkenyl mit 3 bis 20 Kohlenstoffatomen, Aryl mit 6 bis 10 Kohlenstoffatomen oder das Aryl, substituiert mit ein bis drei Substituenten, ausgewählt aus der Gruppe, bestehend aus Alkyl mit 1 bis 20 Kohlenstoffatomen, Cycloalkyl mit 5 bis 12 Kohlenstoffatomen und Phenylalkyl mit 7 bis 15 Kohlenstoffatomen; darstellen; und
wenn n 1 ist, T Alkyl mit 1 bis 20 Kohlenstoffatomen, oder das Alkyl, unterbrochen durch ein oder mehrere von -O-, -S-, -SO-, -SO₂-, -CO-, -COO-, -OCO-, -CONR₁₉-, -NR₁₉CO- oder -NR₂₀-, worin R₁₉ und R₂₀ die gleiche Bedeutung wie R₁₅ aufweisen; oder Aryl oder substituiertes Aryl mit der gleichen Definition wie Ar₁; bedeutet;
wenn n 2 ist, T eine direkte Bindung, Alkylen mit 1 bis 12 Kohlenstoffatomen oder das Alkylen, unterbrochen durch ein oder mehrere von -O-, -S-, -SO-, -SO₂-, -CO-, -COO-, -OCO-, -CONR₂₁-, -NR₂₁CO- oder -NR₂₂-, worin R₂₁ und R₂₂ die gleiche Bedeutung wie R₁₅ aufweisen, bedeutet;
wenn n 3 ist, T Alkantriyl mit 3 bis 8 Kohlenstoffatomen darstellt, und
wenn n 4 ist, T Alkantetrayl mit 4 bis 10 Kohlenstoffatomen darstellt.

9. Masse, umfassend
(a) ein organisches Material, das oxidativem, thermischem oder durch aktinische Strahlung hervorgerufenen Abbau unterliegt, und
(b) eine wirksam stabilisierende Menge einer Verbindung der Formel I, wie in Anspruch 1 definiert.

10. Masse nach Anspruch 9, wobei das organische Material ein synthetisches Polymer darstellt.

11. Masse nach Anspruch 10, wobei das synthetische Polymer ein Polyolefin darstellt.

12. Masse nach Anspruch 11, wobei das Polyolefin Polyethylen oder Polypropylen darstellt.

13. Masse nach Anspruch 9, die zusätzlich eine wirksam stabilisierende Menge eines phenolischen Antioxidans und/oder eines gehinderten Amins enthält.

14. Verfahren zur Stabilisierung organischer Materialien gegen oxidativen, thermischen oder durch aktinische Strahlung hervorgerufenem Abbau, umfassend Versetzen der Materialien mit mindestens einer Verbindung der Formel I nach Anspruch 1.

15. Verwendung einer Verbindung der Formel I nach Anspruch 1 als Stabilisator für organische Materialien gegen oxidativen, thermischen oder durch aktinische Strahlung hervorgerufenem Abbau.

## Revendications

1. Composé qui est une 1-hydroxy-2,6-diaryl-4-acyloxypipéridine ou une 1-hydroxy-2,6-diaryl-4-acylaminopipéridine substituées de formule I 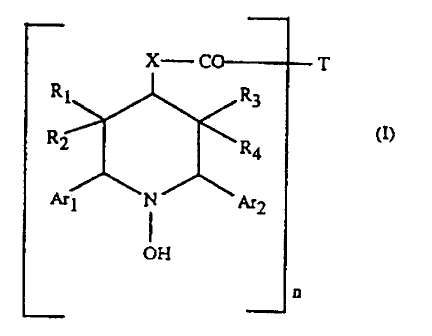 dans laquelle
n vaut 1, 2, 3 ou 4;
X est un radical -O- ou -NE-
E est un atome d'hydrogène, un groupement alkyle de 1 à 20 atomes de carbone ou cycloalkyle de 5 à 12 atomes de carbone,
Ar₁ et Ar₂ représentent indépendamment un groupement aryle de 6 à 10 atomes de carbone; ou ledit groupement aryle substitué par un à trois substituants choisis dans le groupe formé par le groupement alkyle de 1 à 20 atomes de carbone; cycloalkyle de 5 à 12 atomes de carbone; phénylalkyle de 7 à 15 atomes de carbone; -COOR₅ où R₅ est un atome d'hydrogène ou un groupement alkyle de 1 à 20 atomes de carbone; -COR₆ où R₆ est un groupement alkyle de 1 à 20 atomes de carbone; -NR₇R₈ où R₇ et R₈ représentent indépendamment un atome d'hydrogène ou un groupement alkyle de 1 à 20 atomes de carbone; -SR₉ où R₉ est un groupement aryle de 6 à 10 atomes de carbone ou alkyle de 1 à 20 atomes de carbone; -OH; -OCH₃; -CN; -CF₃; -NO₂; -F; -Cl; -Br et -I;
R₁, R₂ R₃ et R₄ représentent indépendamment un atome d'hydrogène; un groupement alkyle linéaire ou ramifié de 1 à 30 atomes de carbone; ou ledit groupement alkyle est terminé par -OR₁₀, -NR₁₁R₁₂, -SR₁₃, -COOR₁₄ ou -CONR₁₅R₁₆, où R₁₀, R₁₁, R₁₂, R₁₃, et R₁₄ représentent indépendamment un groupement alkyle de 1 à 20 atomes de carbone ou alcényle de 3 à 18 atomes de carbone, et R₁₅ et R₁₆ représentent indépendamment un atome d'hydrogène ou ont la même signification que R₁₀; ou ledit groupement alkyle est interrompu par un ou plusieurs radicaux -O-, -S-, -SO-, -SO₂-, -CO-, -COO-, -OCO-, -CONR₁₇-, -NR₁₇CO- ou -NR₁₈ où R₁₇ et R₁₈ ont la même signification que R₁₅; alcényle de 3 à 20 atomes de carbone; aryle de 6 à 10 atomes de carbone; ou ledit groupement aryle substitué par un à trois substituants choisis dans le groupe formé par un groupement alkyle de 1 à 20 atomes de carbone, cycloalkyle de 5 à 12 atomes de carbone, et phénylalkyle de 7 à 15 atomes de carbone; et
lorsque n vaut 1, T est un groupement alkyle de 1 à 20 atomes de carbone ou ledit groupement alkyle est interrompu par un ou plusieurs radicaux -O-, -S-, -SO-, -SO₂-, -CO-, -COO-, -OCO-, -CONR₁₉-, -NR₁₉CO- ou -NR₂₀- où R₁₉ et R₂₀ ont la même signification que R₁₅; ou aryle ou aryle substitué ayant la même définition que Ar₁;
lorsque n vaut 2, T est une liaison directe; un groupement alkylène de 1 à 12 atomes de carbone, ou ledit groupement alkylène interrompu par un ou plusieurs radicaux -O-, -S-, -SO-, -SO₂-, -CO-, -COO-, -OCO-, -CONR₂₁, -NR₂₁CO- ou -NR₂₂- où R₂₁ et R₂₂ ont la même signification que R₁₅;
lorsque n vaut 3, T est un groupement alcanetriyle de 3 à 8 atomes de carbone; et
n vaut 4, T est un groupement alcanetétrayle de 4 à 10 atomes de carbone.

2. Composé de formule I selon la revendication 1, dans lequel
E est un atome d'hydrogène ou un groupement alkyle de 1 à 20 atomes de carbone;
Ar₁ et Ar₂ représentent indépendamment un groupement phényle; ou phényle substitué par un à trois substituants choisis dans le groupe formé d'un groupement alkyle de 1 à 12 atomes de carbone; cycloalkyle de 5 à 7 atomes de carbone; -COOH; -COO-CH₃; -COO-C₂H₅; -CO-CH₃; -CO-C₂H₅; -NH₂; -OH; et -OCH₃;
R₁, R₂, R₃ et R₄ représentent indépendamment un atome d'hydrogène, un groupement méthyle ou éthyle, et lorsque n vaut 1, T est un groupement alkyle de 1 à 20 atomes de carbone ou ledit groupement alkyle interrompu par un ou plusieurs radicaux -O-, -S-, -SO-, -SO₂-, -CO-, -COO-, -OCO- ou phénylène;
lorsque n vaut 2, T est une liaison directe, un groupement alkylène de 1 à 12 atomes de carbone, ou ledit groupement alkylène interrompu par un ou plusieurs radicaux -O-, -S-, -SO-, -SO₂-, -CO-, -COO-, -OCO-;
lorsque n vaut 3, T est un groupement alcanetriyle de 3 à 8 atomes de carbone; et
lorsque n vaut 4, T est un groupement alcanetétrayle de 4 à 10 atomes de carbone.

3. Composé selon la revendication 2, dans lequel Ar₁ et Ar₂ sont identiques et chacun est un groupement phényle ou phényle substitué par un méthyle.

4. Composé selon la revendication 2, dans lequel n vaut 1 ou 2.

5. Composé selon la revendication 2, dans lequel chacun de R₁, R₂, R₃ et R₄ est un atome d'hydrogène, ou R₁ est un groupement méthyle, et R₂, R₃ et R₄ sont des atomes d'hydrogène; ou R₁ et R₃ sont des groupements méthyle et R₂ et R₄ sont des atomes d'hydrogène.

6. Composé selon la revendication 2, dans lequel n vaut 1 ou 2; E est un atome d'hydrogène; chacun de Ar₁ et Ar₂ sont des groupements phényle; chacun de R₁, R₂, R₃ et R₄ est un atome d'hydrogène; ou R₁ est un groupement méthyle, et R₂, R₃ et R₄ sont des atomes d'hydrogène; ou R₁ et R₃ sont des groupements méthyle, et R₂ et R₄ sont des atomes d'hydrogène; et
lorsque n vaut 1, T est un groupement alkyle de 7 à 17 atomes de carbone; et
lorsque n vaut 2, T est un groupement alkylène de 2 à 10 atomes de carbone.

7. Composé selon la revendication 6, dans lequel X est un radical -O-.

8. Composé qui est une 2,6-diaryl-4-acyloxypipéridine ou une 2,6-diaryl-4-acylaminopipéridine de formule II 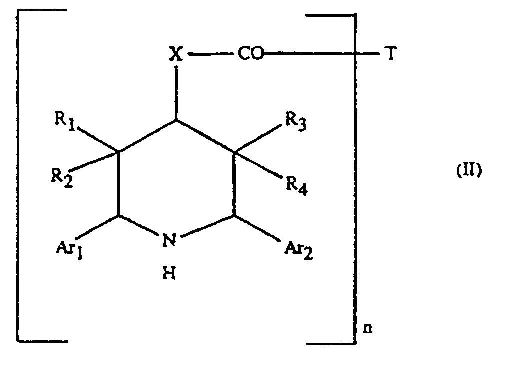 dans laquelle n vaut 1, 2, 3 ou 4;
X est un radical -O- ou -NE-,
E est un atome d'hydrogène, un groupement alkyle de 1 à 20 atomes de carbone ou cycloalkyle de 5 à 12 atomes de carbone;
Ar₁ et Ar₂ sont indépendamment un groupement aryle de 6 à 10 atomes de carbone; ou ledit groupement aryle substitué par un à trois substituants choisis dans le groupe formé d'un groupement alkyle de 1 à 20 atomes de carbone; cycloalkyle de 5 à 12 atomes de carbone; phénylalkyle de 7 à 15 atomes de carbone; -COOR₅ où R₅ est un atome d'hydrogène ou un groupement alkyle de 1 à 20 atomes de carbone; -COR₆ où R₆ est un groupement alkyle de 1 à 20 atomes de carbone; -NR₇R₈ où R₇ et R₈ représentent indépendamment un atome d'hydrogène ou un groupement alkyle de 1 à 20 atomes de carbone; -SR₉ où R₉ est un groupement aryle de 7 à 10 atomes de carbone ou un groupement alkyle de 1 à 20 atomes de carbone; -OH; -OCH₃; -CN; -CF₃; -NO₂; -F; -Cl; -Br et -I;
R₁, R₂, R₃ et R₄ représentent indépendamment un atome d'hydrogène; un groupement alkyle linéaire ou ramifié de 1 à 30 atomes de carbone; ou ledit groupement alkyle terminé par -OR₁₀, -NR₁₁R₁₂-, -SR₁₃, -COOR₁₄ ou -CONR₁₅R₁₆, où R₁₀, R₁₁, R₁₂, R₁₃ et R₁₄ représentent indépendamment un groupement alkyle de 1 à 20 atomes de carbone ou alcényle de 3 à 18 atomes de carbone, et R₁₅ et R₁₆ représentent indépendant un atome d'hydrogène ou ont la même signification que R₁₀; ou ledit groupement alkyle interrompu par un ou plusieurs radicaux -O-, -S-, -SO-, -SO₂-, -CO-, -COO-, -OCO-, -CONR₁₇-, -NR₁₇CO- ou -NR₁₈ où R₁₇ et R₁₈ ont la même signification que R₁₅; alcényle de 3 à 12 atomes de carbone; aryle de 6 à 10 atomes de carbone; ou ledit groupement aryle substitué par un à trois substituants choisis dans le groupe formé des groupements alkyle de 1 à 20 atomes de carbone, cycloakyle de 5 à 12, et phénylalkyle de 7 à 15 atomes de carbone; et
lorsque n vaut 1, T est un groupement alkyle de 1 à 20 atomes de carbone ou ledit groupement alkyle interrompu par un ou plusieurs radicaux -O-, -S-, -SO-, -SO₂-, -CO-,
-COO-, -OCO-, -CONR₁₉-, -NR₁₉CO- ou -NR₂₀- où R₁₉ et R₂₀ ont la même signification que R₁₅; ou aryle ou aryle substitué ayant la même définition que Ar₁;
lorsque n vaut 2, T est une liaison directe; un groupement alkylène de 1 à 12 atomes de carbone, ou ledit groupement alkylène interrompu par un ou plusieurs radicaux -O-, -S-, -SO-, -SO₂-, -CO-, -COO-, -OCO-, -CONR₂₁-, -NR₂₁CO- ou -NR₂₂- où R₂₁ et R₂₂ ont la même signification que R₁₅;
lorsque n vaut 3, T est un groupement alcanetriyle de 3 à 8 atomes de carbone; et
lorsque n vaut 4, T est un groupement alcanetétrayle de 4 à 10 atomes de carbone.

9. Composition qui comprend
(a) une matière organique soumise à une dégradation par oxydation, thermique ou actinique, et
(b) une quantité stabilisante efficace d'un composé de formule I selon la revendication 1.

10. Composition selon la revendication 9, dans laquelle la matière organique est un polymère de synthèse.

11. Composition selon la revendication 10, dans laquelle le polymère de synthèse est une polyoléfine.

12. Composition selon la revendication 11, dans laquelle la polyoléfine est le polyéthylène ou le polypropylène.

13. Composition selon la revendication 9 qui contient en plus une quantité stabilisante efficace d'un antioxydant phénolique et/ou une amine encombrée.

14. Procédé de stabilisation de matières organiques contre une dégradation par oxydation, thermique ou actinique, qui consiste à incorporer dans lesdites matières au moins un composé de formule I selon la revendication 1.

15. Utilisation d'un composé de formule I selon la revendication 1 en tant qu'agent stabilisant pour des matières organiques contre une dégradation par oxydation, thermique ou actinique.
